(19) Europäisches Patentamt
European Patent Office
Office européen des brevets

(11) **EP 4 800 014 A1**

(12) **EUROPEAN PATENT APPLICATION**
published in accordance with Art. 153(4) EPC

(43) Date of publication:
**02.09.2026 Bulletin 2026/36**

(21) Application number: **24881631.6**

(22) Date of filing: **23.10.2024**

(51) International Patent Classification (IPC):
***C07D 471/04*** *(2006.01)* ***C07D 407/04*** *(2006.01)*
***A61K 31/35*** *(2006.01)* ***A61K 31/41*** *(2006.01)*
***A61K 31/435*** *(2006.01)* ***A61K 31/4375*** *(2006.01)*
***A61K 31/495*** *(2006.01)* ***A61K 31/519*** *(2006.01)*
***A61K 31/535*** *(2006.01)* ***A61P 35/00*** *(2006.01)*
***A61P 37/00*** *(2006.01)*

(52) Cooperative Patent Classification (CPC):
**A61K 31/35; A61K 31/41; A61K 31/435; A61K 31/4375; A61K 31/495; A61K 31/519; A61K 31/535; A61P 35/00; A61P 37/00; C07D 407/04; C07D 471/04**

(86) International application number:
**PCT/CN2024/126642**

(87) International publication number:
**WO 2025/087267 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 CN 202311374950**

(71) Applicant: **Tuojie Biotech (Shanghai) Co., Ltd.**
**Shanghai 201203 (CN)**

(72) Inventors:
- **ZHU, Guodong**
  **Shanghai 201203 (CN)**
- **QIAO, Peng**
  **Shanghai 201203 (CN)**
- **LI, Yunfei**
  **Shanghai 201203 (CN)**

(74) Representative: **Dragotti & Associati S.P.A.**
**Via Nino Bixio, 7**
**20129 Milano (MI) (IT)**

(54) **THIAZOLYL-CONTAINING PIPERIDINOPYRIMIDINE DERIVATIVE, PREPARATION METHOD THEREFOR AND MEDICAL USE THEREOF**

(57) The present disclosure relates to a thiazolyl-containing piperidinopyrimidine derivative, a preparation method therefor and a medical use thereof, and specifically relates to a compound represented by formula (I') or a pharmaceutically acceptable salt thereof, and a use of the compound as a CDK7 inhibitor in the treatment of diseases or conditions related to CDK7 activity, wherein each group in the formula (I') is as defined in the description.

EP 4 800 014 A1

## Description

### TECHNICAL FIELD

The present disclosure pertains to the field of pharmaceuticals and relates to a thiazolyl-containing piperidinopyrimidine derivative, a preparation method therefor, and pharmaceutical use thereof.

### BACKGROUND

Cyclin-dependent kinases (CDKs) represent an important category of kinases and play a crucial role in the division and proliferation of cancer cells and the transcriptional regulation of oncogenes. To date, over 20 subtypes of cyclin-dependent kinases (CDKs) have been identified. Given the sequence and structural similarity in the kinase domains of CDK family members, achieving selective and precise regulation of each of the subtypes remains an important challenge.

Cyclin-dependent kinase 7 (CDK7) is a special member of the CDK family that has a dual function in the regulation of cell division and transcription. CDK7 binds to cyclin H and MAT1 to form a trimeric cyclin-activating kinase (CAK). This kinase phosphorylates CDKs involved in cell cycle control (including CDK1, CDK2, CDK4, and CDK6) to activate the activity of the corresponding CDKs, thereby regulating the cell cycle. CDK7 also acts as a component of the common transcription factor II H (TFIIH) and participates in the auxiliary regulation of transcription. It is involved in the initiation of transcription through phosphorylation of the Rbp1 subunit of RNA polymerase II (RNAPII) and can regulate transcriptional elongation by phosphorylating the CDK9 complex.

A hallmark of cancer is uncontrolled cell proliferation and transcriptional dysregulation. Therefore, CDK7 inhibitors that simultaneously inhibit transcription and the cell cycle process are a theoretically and relatively feasible target for cancer treatment. Currently, no drugs that selectively regulate this target are available on the market. The inventors envision developing a highly selective CDK7 inhibitor to treat diseases associated with CDK7 activity.

Patent applications that disclose CDK7 inhibitors include WO2016058544, WO2018013867, WO2019143719, WO2019143730, WO2019099298, WO2020093006, WO2020093011, WO2022064009A, etc.

### SUMMARY

The present disclosure provides a compound represented by formula (I') or a pharmaceutically acceptable salt thereof:

(I') ,

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;

m is selected from the group consisting of 0, 1, 2, 3, and 4;

$L_1$ is selected from the group consisting of a linking bond and $C_{1-3}$ alkylene, wherein the $C_{1-3}$ alkylene is optionally substituted with one or more $R^B$, and $R^B$ is selected from the group consisting of deuterium, halogen, hydroxy, oxo,

$C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, hydroxy, halogen, $C_{1-6}$ alkyl, $C_{1-6}$ haloalkyl, and deuterated $C_{1-6}$ alkyl;

$R^2$, $R^3$, $R^4$, $R^5$, and $R^6$ are each independently selected from the group consisting of hydrogen and deuterium;

$R^7$ is $C_{1-3}$ alkyl, and the $C_{1-3}$ alkyl is optionally substituted with one or more deuterium atoms;

$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;

ring A is selected from the group consisting of phenyl and 5- to 6-membered heteroaryl;

$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;

n is selected from the group consisting of 0, 1, 2, 3, and 4;

$R^9$ is hydrogen or deuterium;

$L_3$ is $C_{1-3}$ alkylene, wherein the $C_{1-3}$ alkylene is optionally substituted with one or more $R^E$, and $R^E$ is deuterium;

$R^{10}$ and $R^{11}$ are each independently $C_{1-3}$ alkyl, wherein the $C_{1-3}$ alkyl is optionally substituted with one or more $R^F$, and $R^F$ is deuterium.

The present disclosure provides a compound represented by formula (I) or a pharmaceutically acceptable salt thereof:

(I)

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;

m is selected from the group consisting of 0, 1, 2, 3, and 4;

$L_1$ is selected from the group consisting of a linking bond and $C_{1-3}$ alkylene, wherein the $C_{1-3}$ alkylene is optionally substituted with one or more $R^B$, and $R^B$ is selected from the group consisting of deuterium, halogen, hydroxy, oxo, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently, optionally, and further substituted with one or more $R^{1B}$ and $R^{1B}$ is selected from the group consisting of deuterium, halogen, $C_{1-6}$ alkyl, $C_{1-6}$ haloalkyl, and deuterated $C_{1-6}$ alkyl;

$R^2$, $R^3$, $R^4$, $R^5$, and $R^6$ are each independently selected from the group consisting of hydrogen and deuterium;

$R^7$ is $C_{1-3}$ alkyl, and the $C_{1-3}$ alkyl is optionally substituted with one or more deuterium atoms;

$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;
ring A is selected from the group consisting of phenyl and 5- to 6-membered heteroaryl;
$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;
n is selected from the group consisting of 0, 1, 2, 3, and 4;
$R^9$ is hydrogen or deuterium;
$L_3$ is $C_{1-3}$ alkylene, wherein the $C_{1-3}$ alkylene is optionally substituted with one or more $R^E$, and $R^E$ is deuterium;
$R^{10}$ and $R^{11}$ are each independently $C_{1-3}$ alkyl, wherein the $C_{1-3}$ alkyl is optionally substituted with one or more $R^F$, and $R^F$ is deuterium.

The present disclosure provides a compound represented by formula (I'-A) or a pharmaceutically acceptable salt thereof:

(I'-A) ,

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;
m is selected from the group consisting of 0, 1, 2, 3, and 4;
$L_1$ is $C_{1-3}$ alkylene, wherein the $C_{1-3}$ alkylene is substituted with one or more $R^B$, and $R^B$ is $C_{1-6}$ alkyl, wherein the $C_{1-6}$ alkyl is substituted with one or more $R^{1B}$, and $R^{1B}$ is hydroxy;
$R^2$, $R^3$, $R^4$, $R^5$, and $R^6$ are each independently selected from the group consisting of hydrogen and deuterium;
$R^7$ is $C_{1-3}$ alkyl, and the $C_{1-3}$ alkyl is optionally substituted with one or more deuterium atoms;
$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;
ring A is selected from the group consisting of phenyl and 5- to 6-membered heteroaryl;
$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl,

and 3- to 6-membered heterocycloalkyl are each independently and optionally substituted with one or more deuterium atoms or halogens;
n is selected from the group consisting of 0, 1, 2, 3, and 4;
$R^9$ is hydrogen or deuterium;
$L_3$ is $C_{1-3}$ alkylene, wherein the $C_{1-3}$ alkylene is optionally substituted with one or more $R^E$, and $R^E$ is deuterium;
$R^{10}$ and $R^{11}$ are each independently $C_{1-3}$ alkyl, wherein the $C_{1-3}$ alkyl is optionally substituted with one or more $R^F$, and $R^F$ is deuterium.

In some embodiments, provided in the present disclosure is the compound represented by formula (I'), formula (I), or formula (I'-A) or the pharmaceutically acceptable salt thereof, wherein $L_2$ is NH.

In some embodiments, provided in the present disclosure is the compound represented by formula (I'), formula (I), or formula (I'-A) or the pharmaceutically acceptable salt thereof, wherein $L_2$ is O.

In some embodiments, provided in the present disclosure is the compound represented by formula (I'), formula (I), or formula (I'-A) or the pharmaceutically acceptable salt thereof, wherein $L_2$ is $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl.

In some embodiments, provided in the present disclosure is the compound represented by formula (I'), formula (I), or formula (I'-A) or the pharmaceutically acceptable salt thereof, wherein $L_2$ is methylene, wherein the methylene is optionally substituted with one or more $R^C$, and $R^C$ is selected from the group consisting of deuterium, halogen, hydroxy, cyano, $C_{1-6}$ alkyl, and $C_{1-6}$ haloalkyl.

In some embodiments, provided in the present disclosure is the compound represented by formula (I'), formula (I), or formula (I'-A) or the pharmaceutically acceptable salt thereof, wherein $L_2$ is methylene.

In some embodiments, the compound represented by formula (I'), formula (I), or formula (I'-A) or the pharmaceutically acceptable salt thereof provided in the present disclosure is a compound represented by formula (II) or a pharmaceutically acceptable salt thereof:

(II)

wherein $R^1$, m, $L_1$, $R^2$, $R^3$, $R^4$, $R^5$, $R^6$, ring A, $R^8$, n, $L_3$, $R^{10}$, and $R^{11}$ are each as defined in the compound represented by formula (I'), formula (I), or formula (I'-A) or the pharmaceutically acceptable salt thereof.

In some embodiments, provided in the present disclosure is the compound represented by formula (I'), (I), (I'-A), or (II) or the pharmaceutically acceptable salt thereof, wherein $L_3$ is selected from the group consisting of methylene and ethylene, wherein the methylene and ethylene are each independently and optionally substituted with one or more $R^E$, and $R^E$ is deuterium.

In some embodiments, provided in the present disclosure is the compound represented by formula (I'), (I), (I'-A), or (II) or the pharmaceutically acceptable salt thereof, wherein $L_3$ is methylene, wherein the methylene is optionally substituted with one or more $R^E$, and $R^E$ is deuterium.

In some embodiments, provided in the present disclosure is the compound represented by formula (I'), (I), (I'-A), or (II) or the pharmaceutically acceptable salt thereof, wherein $L_3$ is methylene, wherein the methylene is substituted with one or more $R^E$, and $R^E$ is deuterium.

In some embodiments, provided in the present disclosure is the compound represented by formula (I'), (I), (I'-A), or (II) or the pharmaceutically acceptable salt thereof, wherein $R^{10}$ is selected from the group consisting of $C_{1-3}$ alkyl (e.g., methyl, ethyl, n-propyl, or isopropyl), wherein the $C_{1-3}$ alkyl is optionally substituted with one or more $R^F$, and $R^F$ is deuterium.

In some embodiments, provided in the present disclosure is the compound represented by formula (I'), (I), (I'-A), or (II) or the pharmaceutically acceptable salt thereof, wherein $R^{10}$ is selected from the group consisting of methyl and ethyl, wherein the methyl and ethyl are each independently and optionally substituted with one or more $R^F$, and $R^F$ is deuterium.

In some embodiments, provided in the present disclosure is the compound represented by formula (I'), (I), (I'-A),

or (II) or the pharmaceutically acceptable salt thereof, wherein $R^{10}$ is methyl.

In some embodiments, provided in the present disclosure is the compound represented by formula (I'), (I), (I'-A), or (II) or the pharmaceutically acceptable salt thereof, wherein $R^{10}$ is $-CD_3$.

In some embodiments, provided in the present disclosure is the compound represented by formula (I'), (I), (I'-A), or (II) or the pharmaceutically acceptable salt thereof, wherein $R^{11}$ is selected from the group consisting of $C_{1-3}$ alkyl (e.g., methyl, ethyl, n-propyl, or isopropyl), wherein the $C_{1-3}$ alkyl is optionally substituted with one or more $R^F$, and $R^F$ is deuterium.

In some embodiments, provided in the present disclosure is the compound represented by formula (I'), (I), (I'-A), or (II) or the pharmaceutically acceptable salt thereof, wherein $R^{11}$ is selected from the group consisting of methyl and ethyl, wherein the methyl and ethyl are each independently and optionally substituted with one or more $R^F$, and $R^F$ is deuterium.

In some embodiments, provided in the present disclosure is the compound represented by formula (I'), (I), (I'-A), or (II) or the pharmaceutically acceptable salt thereof, wherein $R^{11}$ is methyl.

In some embodiments, provided in the present disclosure is the compound represented by formula (I'), (I), (I'-A), or (II) or the pharmaceutically acceptable salt thereof, wherein $R^{11}$ is $-CD_3$.

In some embodiments, provided in the present disclosure is the compound represented by formula (I'), (I), (I'-A), or (II) or the pharmaceutically acceptable salt thereof, wherein ring A is phenyl.

In some embodiments, provided in the present disclosure is the compound represented by formula (I'), (I), (I'-A), or (II) or the pharmaceutically acceptable salt thereof, wherein ring A is 5- to 6-membered heteroaryl.

In some embodiments, provided in the present disclosure is the compound represented by formula (I'), (I), (I'-A), or (II) or the pharmaceutically acceptable salt thereof, wherein ring A is pyridine.

In some embodiments, provided in the present disclosure is the compound represented by formula (I'), (I), (I'-A), or (II) or the pharmaceutically acceptable salt thereof, wherein ring A is pyrazole.

In some embodiments, provided in the present disclosure is the compound represented by formula (I'), (I), (I'-A), or (II) or the pharmaceutically acceptable salt thereof, wherein ring A is imidazole.

In some embodiments, provided in the present disclosure is the compound represented by formula (I'), (I), (I'-A), or (II) or the pharmaceutically acceptable salt thereof, wherein ring A is thiazole.

In some embodiments, provided in the present disclosure is the compound represented by formula (I'), (I), or (II) or the pharmaceutically acceptable salt thereof, wherein $L_1$ is a linking bond.

In some embodiments, provided in the present disclosure is the compound represented by formula (I'), (I), or (II) or the pharmaceutically acceptable salt thereof, wherein $L_1$ is $C_{1-3}$ alkylene, wherein the $C_{1-3}$ alkylene is optionally substituted with one or more $R^B$, and $R^B$ is selected from the group consisting of deuterium, halogen, hydroxy, oxo, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, halogen, $C_{1-6}$ alkyl, $C_{1-6}$ haloalkyl, and deuterated $C_{1-6}$ alkyl.

In some embodiments, provided in the present disclosure is the compound represented by formula (I'), (I), or (II) or the pharmaceutically acceptable salt thereof, wherein $L_1$ is methylene, wherein the methylene is substituted with one or more $R^B$, and $R^B$ is selected from the group consisting of deuterium, halogen, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, halogen, $C_{1-6}$ alkyl, $C_{1-6}$ haloalkyl, and deuterated $C_{1-6}$ alkyl.

In some embodiments, provided in the present disclosure is the compound represented by (I'), (I), or (II) or the pharmaceutically acceptable salt thereof, wherein $L_1$ is methylene, wherein the methylene is substituted with one or more $R^B$, and $R^B$ is selected from the group consisting of deuterium, halogen, $C_{1-3}$ alkyl, 3- to 6-membered cycloalkyl, and 3- to 6-membered heterocycloalkyl, wherein the $C_{1-3}$ alkyl, 3- to 6-membered cycloalkyl, and 3- to 6-membered heterocycloalkyl are each independently and optionally substituted with one or more $R^{1B}$ and $R^{1B}$ is selected from the group consisting of deuterium and halogen.

In some embodiments, provided in the present disclosure is the compound represented by (I'), (I), or (II) or the pharmaceutically acceptable salt thereof, wherein $L_1$ is methylene, wherein the methylene is substituted with one or more $R^B$, and $R^B$ is selected from the group consisting of $C_{1-3}$ alkyl, 3- to 6-membered cycloalkyl, and 3- to 6-membered heterocycloalkyl, wherein the $C_{1-3}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium and halogen.

In some embodiments, provided in the present disclosure is the compound represented by formula (I'), (I), or (II) or the pharmaceutically acceptable salt thereof, wherein $L_1$ is selected from the group consisting of methylene.

In some embodiments, provided in the present disclosure is the compound represented by formula (II) or the pharmaceutically acceptable salt thereof, wherein ring A is phenyl;

$R^2$, $R^3$, $R^4$, $R^5$, and $R^6$ are each independently hydrogen;
$R^{10}$ is selected from the group consisting of methyl and $-CD_3$;
$R^{11}$ is selected from the group consisting of methyl and $-CD_3$;
$L_1$ is methylene, wherein the methylene is substituted with one or more $R^B$, and $R^B$ is selected from the group consisting of deuterium, halogen, methyl, ethyl, cyclopropyl, and cyclobutyl, wherein the methyl, ethyl, cyclopropyl, and cyclobutyl are each independently and optionally substituted with one or more $R^{1B}$ and $R^{1B}$ is selected from the group consisting of deuterium and halogen.

In some embodiments, provided in the present disclosure is the compound represented by formula (I'), (I), (I'-A), or (II) or the pharmaceutically acceptable salt thereof, wherein $R^8$ is selected from the group consisting of deuterium, halogen, hydroxy, cyano, carboxyl, $C_{1-6}$ alkyl, and $C_{1-6}$ alkoxy, wherein the $C_{1-6}$ alkyl and $C_{1-6}$ alkoxy are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;
n is selected from the group consisting of 0, 1, 2, and 3.

In some embodiments, provided in the present disclosure is the compound represented by formula (I'), (I), (I'-A), or (II) or the pharmaceutically acceptable salt thereof, wherein $R^8$ is selected from the group consisting of deuterium, halogen, hydroxy, cyano, carboxyl, methyl, ethyl, isopropyl, n-propyl, n-butyl, methoxy, ethoxy, isopropoxy, and n-butoxy, wherein the methyl, ethyl, isopropyl, n-propyl, n-butyl, methoxy, ethoxy, isopropoxy, and n-butoxy are each independently and optionally substituted with one or more $R^D$, and $R^D$ is selected from the group consisting of deuterium, halogen, hydroxy, methyl, ethyl, ethoxy, and methoxy, wherein the methyl, ethyl, ethoxy, and methoxy are each independently and optionally substituted with one or more deuterium atoms or halogens;
n is selected from the group consisting of 0, 1, 2, and 3.

In some embodiments, provided in the present disclosure is the compound represented by formula (I'), (I), (I'-A), or (II) or the pharmaceutically acceptable salt thereof, wherein $R^8$ is selected from the group consisting of deuterium, halogen, methyl, ethyl, methoxy, and ethoxy, wherein the methyl, ethyl, methoxy, and ethoxy are each independently and optionally substituted with one or more $R^D$, and $R^D$ is selected from the group consisting of deuterium, halogen, and hydroxy;
n is selected from the group consisting of 0, 1, 2, and 3.

In some embodiments, provided in the present disclosure is the compound represented by formula (I'), (I), (I'-A), or (II) or the pharmaceutically acceptable salt thereof, wherein $R^1$ is selected from the group consisting of deuterium, cyano, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more $R^A$, and $R^A$ is selected from the group consisting of deuterium, halogen, hydroxy, amino, oxo, $C_{1-6}$ alkyl, and $C_{1-6}$ alkoxy, wherein the $C_{1-6}$ alkyl and $C_{1-6}$ alkoxy are each independently and optionally substituted with one or more deuterium atoms or halogens;
m is selected from the group consisting of 0, 1, 2, and 3.

In some embodiments, provided in the present disclosure is the compound represented by formula (I'), (I), (I'-A), or (II) or the pharmaceutically acceptable salt thereof, wherein $R^1$ is selected from the group consisting of $C_{1-6}$ alkyl and 3- to 6-membered cycloalkyl, wherein the $C_{1-6}$ alkyl and 3- to 6-membered cycloalkyl are each independently and optionally substituted with one or more $R^A$, and $R^A$ is selected from the group consisting of deuterium, halogen, hydroxy, amino, oxo, methyl, ethyl, methoxy, and ethoxy;
m is selected from the group consisting of 0, 1, 2, and 3.

In some embodiments, provided in the present disclosure is the compound represented by formula (I'), (I), (I'-A), or (II) or the pharmaceutically acceptable salt thereof, wherein $R^1$ is selected from the group consisting of methyl, ethyl, n-propyl, isopropyl, n-butyl, isobutyl, cyclopropyl, cyclobutyl, and cyclopentyl, wherein the methyl, ethyl, n-propyl, isopropyl, n-butyl, isobutyl, cyclopropyl, cyclobutyl, and cyclopentyl are each independently and optionally substituted with one or more $R^A$, and $R^A$ is selected from the group consisting of deuterium, halogen, and hydroxy;
m is selected from the group consisting of 0, 1, 2, and 3.

In some embodiments, provided in the present disclosure is the compound represented by formula (I'), (I), or (II) or the pharmaceutically acceptable salt thereof, wherein $L_1$ is methylene, wherein the methylene is substituted with one or more $R^B$, and $R^B$ is deuterium or $C_{1-6}$ alkyl.

In some embodiments, provided in the present disclosure is the compound represented by formula (I'), (I), or (II) or the pharmaceutically acceptable salt thereof, wherein

is

.

In some embodiments, provided in the present disclosure is the compound represented by formula (I'), (I), or (II) or the pharmaceutically acceptable salt thereof, wherein

is

.

In some embodiments, provided in the present disclosure is the compound represented by formula (I'), (I), or (II) or the pharmaceutically acceptable salt thereof, wherein

is

.

In some embodiments, provided in the present disclosure is the compound represented by formula (I'), (I), or (II) or the pharmaceutically acceptable salt thereof, wherein

is

.

In some embodiments, provided in the present disclosure is the compound represented by formula (I'), (I), or (II) or the pharmaceutically acceptable salt thereof, wherein

is

In some embodiments, provided in the present disclosure is the compound represented by formula (I'), (I), or (II) or the pharmaceutically acceptable salt thereof, wherein

is

In some embodiments, provided in the present disclosure is the compound represented by formula (I'), (I), or (II) or the pharmaceutically acceptable salt thereof, wherein

is

In some embodiments, provided in the present disclosure is the compound represented by formula (I'), (I), or (II) or the pharmaceutically acceptable salt thereof, wherein

is

In some embodiments, the compound represented by formula (I'), (I), or (II) or the pharmaceutically acceptable salt thereof provided in the present disclosure is a compound represented by formula (III-1) or a pharmaceutically acceptable salt thereof:

(III-1)

wherein $R^8$, n, $R^1$, and m are as defined in the compound represented by formula (I) or the pharmaceutically acceptable salt thereof;
$R^B$ is selected from the group consisting of hydrogen, deuterium, methyl, ethyl, cyclopropyl, and cyclobutyl, wherein the methyl, ethyl, cyclopropyl, and cyclobutyl are each independently and optionally substituted with one or more $R^{1B}$, and $R^{1B}$ is selected from the group consisting of deuterium and halogen.

In some embodiments, the compound represented by formula (I'), (I), or (II) or the pharmaceutically acceptable salt thereof provided in the present disclosure is a compound represented by formula (III-2) or a pharmaceutically acceptable salt thereof:

(III-2)

wherein $R^8$, n, $R^1$, and m are as defined in the compound represented by formula (I) or the pharmaceutically acceptable salt thereof;
$R^B$ is selected from the group consisting of hydrogen, deuterium, methyl, ethyl, cyclopropyl, and cyclobutyl, wherein the methyl, ethyl, cyclopropyl, and cyclobutyl are each independently and optionally substituted with one or more $R^{1B}$, and $R^{1B}$ is selected from the group consisting of deuterium and halogen.

In some embodiments, the compound represented by formula (I'), (I), or (II) or the pharmaceutically acceptable salt thereof provided in the present disclosure is a compound represented by formula (III-3) or a pharmaceutically acceptable salt thereof:

(III-3)

wherein $R^8$, n, $R^1$, and m are as defined in the compound represented by formula (I) or the pharmaceutically acceptable salt thereof;

$R^B$ is selected from the group consisting of hydrogen, deuterium, methyl, ethyl, cyclopropyl, and cyclobutyl, wherein the methyl, ethyl, cyclopropyl, and cyclobutyl are each independently and optionally substituted with one or more $R^{1B}$, and $R^{1B}$ is selected from the group consisting of deuterium and halogen.

In some embodiments, the compound represented by formula (I'), (I), or (II) or the pharmaceutically acceptable salt thereof provided in the present disclosure is a compound represented by formula (III-4) or a pharmaceutically acceptable salt thereof:

(III-4)

wherein $R^8$, n, $R^1$, and m are as defined in the compound represented by formula (I) or the pharmaceutically acceptable salt thereof;

$R^B$ is selected from the group consisting of hydrogen, deuterium, methyl, ethyl, cyclopropyl, and cyclobutyl, wherein the methyl, ethyl, cyclopropyl, and cyclobutyl are each independently and optionally substituted with one or more $R^{1B}$, and $R^{1B}$ is selected from the group consisting of deuterium and halogen.

In an optional embodiment, provided in the present disclosure is the compound represented by formula (III-1), (III-2), (III-3), or (III-4) or the pharmaceutically acceptable salt thereof, wherein $R^8$ is selected from the group consisting of deuterium, halogen, methyl, ethyl, methoxy, and ethoxy, wherein the methyl, ethyl, methoxy, and ethoxy are each independently and optionally substituted with one or more $R^D$, and $R^D$ is selected from the group consisting of deuterium, halogen, and hydroxy;

$R^1$ is selected from the group consisting of methyl, ethyl, n-propyl, isopropyl, n-butyl, isobutyl, cyclopropyl, cyclobutyl, and cyclopentyl, wherein the methyl, ethyl, n-propyl, isopropyl, n-butyl, isobutyl, cyclopropyl, cyclobutyl, and cyclopentyl are each independently and optionally substituted with one or more $R^A$, and $R^A$ is selected from the group consisting of deuterium and halogen;

m and n are each independently selected from the group consisting of 0, 1, 2, and 3.

In an optional embodiment, provided in the present disclosure is the compound represented by formula (III-1), (III-2), (III-3), or (III-4) or the pharmaceutically acceptable salt thereof, wherein $R^B$ is selected from the group consisting of methyl and cyclopropyl, wherein the methyl and cyclopropyl are each independently and optionally substituted with one or more $R^{1B}$, and $R^{1B}$ is deuterium;

$R^1$ is selected from the group consisting of methyl and cyclopropyl, wherein the methyl and cyclopropyl are each independently and optionally substituted with one or more $R^A$, and $R^A$ is selected from the group consisting of deuterium and halogen;

m is selected from the group consisting of 0, 1, 2, and 3;

n is 0.

The compound represented by formula (I'), (I), (I'-A), (II), (III-1), (III-2), (III-3), or (III-4) or the pharmaceutically acceptable salt thereof provided in the present disclosure is a compound shown in table a or a pharmaceutically acceptable salt thereof:

**Table a**

In another aspect, the present disclosure provides an isotopically substituted form of the compound represented by formula (I'), (I), (I'-A), (II), (III-1), (III-2), (III-3), or (III-4) or the pharmaceutically acceptable salt thereof or the compound shown in Table a. In an optional embodiment, the isotopically substituted form is a deuterated form.

The present disclosure further provides a pharmaceutical composition comprising at least one of the compound represented by formula (I'), (I), (I'-A), (II), (III-1), (III-2), (III-3), or (III-4) or the pharmaceutically acceptable salt thereof, the compound shown in Table a, or the isotopically substituted form, and a pharmaceutically acceptable excipient.

In some embodiments, a unit dose of the pharmaceutical composition is 0.001 mg-1000 mg.

In certain embodiments, the pharmaceutical composition comprises 0.01-99.99% of an aforementioned compound or the pharmaceutically acceptable salt thereof based on the total weight of the composition. In certain embodiments, the pharmaceutical composition comprises 0.1-99.9% of an aforementioned compound or the pharmaceutically acceptable salt thereof. In certain embodiments, the pharmaceutical composition comprises 0.5%-99.5% of an aforementioned compound or the pharmaceutically acceptable salt thereof. In certain embodiments, the pharmaceutical composition comprises 1%-99% of an aforementioned compound or the pharmaceutically acceptable salt thereof. In certain embodiments, the pharmaceutical composition comprises 2%-98% of an aforementioned compound or the pharmaceutically acceptable salt thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure provides use of the aforementioned compound represented by formula (I'), (I), (I'-A), (II), (III-1), (III-2), (III-3), or (III-4) or the pharmaceutically acceptable salt thereof, the compound shown in Table a, the isotopically substituted form, or the aforementioned pharmaceutical composition in the manufacture of a medicament for treating or preventing a disease or disorder associated with abnormal activity of a serine/threonine kinase.

In another aspect, the present disclosure provides use of the aforementioned compound represented by formula (I'), (I), (I'-A), (II), (III-1), (III-2), (III-3), or (III-4) or the pharmaceutically acceptable salt thereof, the compound shown in

Table a, the isotopically substituted form, or the aforementioned pharmaceutical composition in the manufacture of a medicament for treating and/or preventing a disease or disorder associated with abnormal activity of CDK7.

In an optional embodiment, the disease or disorder associated with abnormal activity of CDK7 is selected from the group consisting of a proliferative disease, an inflammatory disease, an auto inflammatory disease, an autoimmune disease, and an infectious disease. In another aspect, the present disclosure provides use of the aforementioned compound represented by formula (I'), (I), (I'-A), (II), (III-1), (III-2), (III-3), or (III-4) or the pharmaceutically acceptable salt thereof, the compound shown in Table a, the isotopically substituted form, or the aforementioned pharmaceutical composition in the manufacture of a medicament for treating and/or preventing a disease or disorder, wherein the disease or disorder is selected from the group consisting of a proliferative disease, an inflammatory disease, an auto inflammatory disease, an autoimmune disease, and an infectious disease.

In an optional embodiment, the disease or disorder is a proliferative disease.

In an optional embodiment, the proliferative disease is cancer.

In an optional embodiment, the cancer is selected from the group consisting of a hematological tumor and a solid tumor.

In an optional embodiment, the hematological tumor is selected from the group consisting of leukemias, specifically including: chronic lymphocytic leukemia (CLL), acute lymphocytic leukemia (ALL), T-cell acute lymphocytic leukemia (T-ALL), chronic myeloid leukemia (CML), acute myeloid leukemia (AML), etc.

In an optional embodiment, the solid tumor is selected from the group consisting of breast cancer, intestinal cancer, lung cancer, pancreatic cancer, prostate cancer, Ewing sarcoma, osteoma, neuroblastoma, cervical cancer, ovarian cancer, gastric cancer, and liver cancer.

In an optional embodiment, the breast cancer is triple-negative breast cancer.

In an optional embodiment, the breast cancer is ER/PR+ HER2- breast cancer.

In an optional embodiment, the breast cancer is ER/PR+ HER2- breast cancer resistant to a CDK4/6 inhibitor.

In an optional embodiment, the CDK4/6 inhibitor is palbociclib.

In an optional embodiment, the lung cancer is non-small cell lung cancer.

In an optional embodiment, the lung cancer is small cell lung cancer.

In an optional embodiment, the intestinal cancer is colon cancer.

In an optional embodiment, the intestinal cancer is rectal cancer.

In another aspect, the present disclosure provides a method for treating and/or preventing a disease or disorder associated with abnormal activity of a serine/threonine kinase in a patient, comprising administering to the patient a therapeutically effective amount of the compound represented by formula (I'), (I), (I'-A), (II), (III-1), (III-2), (III-3), or (III-4) or the pharmaceutically acceptable salt thereof, the compound shown in Table a, the isotopically substituted form, or the aforementioned pharmaceutical composition.

In another aspect, the present disclosure provides a method for treating and/or preventing a disease or disorder associated with abnormal activity of CDK7 in a patient, comprising administering to the patient a therapeutically effective amount of the compound represented by formula (I'), (I), (I'-A), (II), (III-1), (III-2), (III-3), or (III-4) or the pharmaceutically acceptable salt thereof, the compound shown in Table a, the isotopically substituted form, or the aforementioned pharmaceutical composition.

In an optional embodiment, the disease or disorder associated with abnormal activity of CDK7 is selected from the group consisting of a proliferative disease, an inflammatory disease, an auto inflammatory disease, an autoimmune disease, and an infectious disease. In another aspect, the present disclosure provides a method for treating and/or preventing a disease or disorder in a patient, comprising administering to the patient a therapeutically effective amount of the compound represented by formula (I'), (I), (I'-A), (II), (III-1), (III-2), (III-3), or (III-4) or the pharmaceutically acceptable salt thereof, the compound shown in Table a, the isotopically substituted form, or the aforementioned pharmaceutical composition, wherein the disease or disorder is selected from the group consisting of a proliferative disease, an inflammatory disease, an auto inflammatory disease, an autoimmune disease, and an infectious disease; in an optional embodiment, the disease or disorder is a proliferative disease.

In an optional embodiment, the proliferative disease is cancer.

In an optional embodiment, the cancer is selected from the group consisting of a hematological tumor and a solid tumor.

In an optional embodiment, the hematological tumor is selected from the group consisting of leukemias, specifically including: chronic lymphocytic leukemia (CLL), acute lymphocytic leukemia (ALL), T-cell acute lymphocytic leukemia (T-ALL), chronic myeloid leukemia (CML), acute myeloid leukemia (AML), etc.

In an optional embodiment, the solid tumor is selected from the group consisting of breast cancer, intestinal cancer, lung cancer, pancreatic cancer, prostate cancer, Ewing sarcoma, osteoma, neuroblastoma, cervical cancer, ovarian cancer, gastric cancer, and liver cancer.

In an optional embodiment, the breast cancer is triple-negative breast cancer.

In an optional embodiment, the breast cancer is ER/PR+ HER2- breast cancer.

In an optional embodiment, the breast cancer is ER/PR+ HER2- breast cancer resistant to a CDK4/6 inhibitor.

In an optional embodiment, the CDK4/6 inhibitor is palbociclib.

In an optional embodiment, the lung cancer is non-small cell lung cancer.

In an optional embodiment, the lung cancer is small cell lung cancer.

In an optional embodiment, the intestinal cancer is colon cancer.

In an optional embodiment, the intestinal cancer is rectal cancer.

In another aspect, the present disclosure provides use of the compound represented by formula (I'), (I), (I'-A), (II), (III-1), (III-2), (III-3), or (III-4) or the pharmaceutically acceptable salt thereof, the compound shown in Table a, or the isotopically substituted form as a medicament.

In another aspect, the present disclosure provides use of the compound represented by formula (I'), (I), (I'-A), (II), (III-1), (III-2), (III-3), or (III-4) or the pharmaceutically acceptable salt thereof, the compound shown in Table a, or the isotopically substituted form in the manufacture of an antibody-drug conjugate or a proteolysis targeting chimera.

In another aspect, the present disclosure provides an antibody-drug conjugate comprising the compound represented by formula (I'), (I), (I'-A), (II), (III-1), (III-2), (III-3), or (III-4) or the pharmaceutically acceptable salt thereof, the compound shown in Table a, or the isotopically substituted form.

In another aspect, the present disclosure provides a proteolysis targeting chimera comprising the compound represented by formula (I'), (I), (I'-A), (II), (III-l), (III-2), (III-3), or (III-4) or the pharmaceutically acceptable salt thereof, the compound shown in Table a, or the isotopically substituted form.

The present disclosure further provides a preparation method for the aforementioned compound represented by formula (I'), (I), or (I'-A) or the pharmaceutically acceptable salt thereof:

(I-A) + (I-B) →

comprising a step of catalyzing a reaction of a compound represented by formula (I-A) or a pharmaceutically acceptable salt thereof with a compound represented by formula (I-B) or a pharmaceutically acceptable salt thereof in an alkaline environment with a catalyst selected from the group consisting of carbonyldiimidazole, phosgene, and triphosgene,

wherein $L_2$ is NH;
$R^1$, m, $L_1$, $R^2$, $R^3$, $R^4$, $R^5$, $R^6$, $R^7$, ring A, $R^8$, n, $R^9$, $L_3$, $R^{10}$, and $R^{11}$ are as defined in the compound represented by formula (I'), (I), or (I'-A) or the pharmaceutically acceptable salt thereof.

In an optional embodiment, the condensation reaction takes place in an alkaline environment provided by an inorganic base (sodium hydroxide) or an organic base (e.g., triethylamine, pyridine, piperidine, or N,N-diisopropylethylamine), and the solvent in which the reaction takes place is a common solvent (e.g., DMF, DCM, or DMSO).

The present disclosure further provides a compound represented by formula (I-A) or a pharmaceutically acceptable salt thereof:

(I-A)

wherein $R^1$, m, $L_1$, $R^2$, $R^3$, $R^4$, $R^5$, $R^6$, and $R^7$ are each as defined in the compound represented by formula (I'), (I), or (I'-A) or the pharmaceutically acceptable salt thereof.

The pharmaceutically acceptable salts of the compounds described in the present disclosure are selected from the group consisting of inorganic salts and organic salts. The compounds described in the present disclosure can react with acidic or basic substances to form corresponding salts.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures, and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof fall within the scope of the present disclosure. The compounds of the present disclosure containing asymmetric carbon atoms may be isolated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

In the chemical structures of the compounds of the present disclosure, the bond "" indicates an unspecified configuration; that is, if chiral isomers exist in the chemical structures, the bond "" may be "" or "", or includes both the configurations "" and "" simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization. An example of a lactam-lactim equilibrium is present between A and B as shown below.

All compounds in the present disclosure can be drawn as form A or form B. All tautomeric forms fall within the scope of the present disclosure. The names of the compounds do not exclude any tautomers.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced with a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

Terms and definitions:

"Pharmaceutical composition" refers to a mixture containing one or more of the compounds or the physiologically and pharmaceutically acceptable salts or pro-drugs thereof described herein, and other chemical components, as well as

other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

"Effective amount" or "therapeutically effective amount" as described in the present disclosure includes an amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. An effective amount may be the maximum dose or administration regimen to avoid significant side effects or toxic effects.

The prefix "$C_{u-v}$" indicates that the following group has from u to v carbon atoms. For example, "$C_{1-6}$ alkyl" indicates that the alkyl group has 1 to 6 carbon atoms. Specifically, it may be an alkyl group having 1, 2, 3, 4, 5, or 6 carbon atoms.

The term "alkyl" refers to an unbranched or branched saturated hydrocarbon chain. As used herein, alkyl has 1 to 20 carbon atoms (i.e., $C_{1-20}$ alkyl), 1 to 8 carbon atoms (i.e., $C_{1-8}$ alkyl), 1 to 6 carbon atoms (i.e., $C_{1-6}$ alkyl), or 1 to 4 carbon atoms (i.e., $C_{1-4}$ alkyl). Examples of alkyl groups include methyl, ethyl, propyl, isopropyl, n-butyl, sec-butyl, isobutyl, tert-butyl, pentyl, 2-pentyl, isopentyl, neopentyl, hexyl, 2-hexyl, 3-hexyl, and 3-methylpentyl. When an alkyl residue having a specific number of carbon atoms is named after a chemical name or identified by a molecular formula, all positional isomers having that number of carbon atoms may be included; thus, for example, "butyl" includes n-butyl (i.e., $-(CH_2)_3CH_3$), sec-butyl (i.e., $-CH(CH_3)CH_2CH_3$), isobutyl (i.e., $-CH_2CH(CH_3)_2$), and tert-butyl (i.e., $-C(CH_3)_3$); and "propyl" includes n-propyl (i.e., $-(CH_2)_2CH_3$) and isopropyl (i.e., $-CH(CH_3)_2$).

The term "alkenyl" refers to an alkyl group containing at least one carbon-carbon double bond and having 2 to 20 carbon atoms (i.e., $C_{2-20}$ alkenyl), 2 to 8 carbon atoms (i.e., $C_{2-8}$ alkenyl), 2 to 6 carbon atoms (i.e., $C_{2-6}$ alkenyl), or 2 to 4 carbon atoms (i.e., $C_{2-4}$ alkenyl). Examples of alkenyl groups include ethenyl, propenyl, and butadienyl (including 1,2-butadienyl and 1,3-butadienyl).

The term "alkynyl" refers to an alkyl group containing at least one carbon-carbon triple bond and having 2 to 20 carbon atoms (i.e., $C_{2-20}$ alkynyl), 2 to 8 carbon atoms (i.e., $C_{2-8}$ alkynyl), 2 to 6 carbon atoms (i.e., $C_{2-6}$ alkynyl), or 2 to 4 carbon atoms (i.e., $C_{2-4}$ alkynyl). Examples of "alkynyl" groups include ethynyl, propynyl (e.g., 1-propynyl, 2-propynyl), 3-butynyl, pentynyl, hexynyl, and 1-methylpent-2-ynyl.

The term "cycloalkyl" or "carbocycle" refers to a saturated or partially unsaturated cyclic alkyl group having a single ring or multiple rings (including fused, bridged, and spiro ring systems). The term "cycloalkyl" includes cycloalkenyl (that is, the cyclic group has at least one double bond). As used herein, cyclic alkyl has 3 to 20 ring carbon atoms (i.e., $C_{3-20}$ cycloalkyl), 3 to 12 ring carbon atoms (i.e., $C_{3-12}$ cycloalkyl), 3 to 10 ring carbon atoms (i.e., $C_{3-10}$ cycloalkyl), 3 to 8 ring carbon atoms (i.e., $C_{3-8}$ cycloalkyl), or 3 to 7 ring carbon atoms (i.e., $C_{3-7}$ cycloalkyl), or 3 to 6 ring carbon atoms (i.e., $C_{3-6}$ cycloalkyl). Examples of cycloalkyl groups include cyclopropyl, cyclobutyl, cyclopentyl, cyclohexyl, cyclohexenyl, and cyclohexadienyl. The cycloalkyl ring may be fused to an aryl or heteroaryl ring, wherein the ring attached to the parental structure is cycloalkyl; non-limiting examples include indanyl, tetrahydronaphthyl, benzocycloheptyl, etc.

The term "heterocyclyl" or "heterocycloalkyl" refers to a saturated or unsaturated cycloalkyl group having one or more ring heteroatoms independently selected from the group consisting of nitrogen, oxygen, sulfur, and phosphorus. The term "heterocycloalkyl" includes heterocycloalkenyl groups (i.e., heterocyclyl groups having at least one double bond), bridged-heterocyclyl groups, fused-heterocyclyl groups, and spiro-heterocyclyl groups. A heterocyclyl group may be a single ring or multiple rings, wherein the multiple rings may be fused, bridged, or spiro. Any non-aromatic ring containing at least one heteroatom is considered a heterocyclyl group, regardless of the attachment (that is, binding via a carbon atom or a heteroatom is possible). In addition, the term heterocyclyl is intended to include any non-aromatic ring containing at least one heteroatom, and the ring may be fused to an aryl or heteroaryl ring, regardless of the attachment to the remainder of the molecule. As used herein, heterocyclyl has 3 to 20 ring atoms (i.e., 3- to 20-membered heterocyclyl), 3 to 12 ring atoms (i.e., 3- to 12-membered heterocyclyl), 3 to 10 ring atoms (i.e., 3- to 10-membered heterocyclyl), 3 to 8 ring atoms (i.e., 3- to 8-membered heterocyclyl), 3 to 7 ring atoms (i.e., 3- to 7-membered heterocyclyl), or 3 to 6 ring atoms (i.e., 3- to 6-membered heterocyclyl) and has 1 to 5 ring heteroatoms, 1 to 4 ring heteroatoms, 1 to 3 ring heteroatoms, 1 to 2 ring heteroatoms, or 1 ring heteroatom, and the ring heteroatoms are independently selected from the group consisting of nitrogen, sulfur, phosphorus, and oxygen. Examples of heterocyclyl groups include pyrrolidinyl, imidazolidinyl, oxetanyl, dioxolanyl, azetidinyl, tetrahydrofuranyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, and homopiperazinyl.

Non-limiting examples of "heterocyclyl" groups include:

etc.

The heterocyclyl ring may be fused to an aryl or heteroaryl ring, wherein the ring attached to the parental structure is heterocyclyl; its non-limiting examples include:

etc.

The term "aryl" refers to a 6- to 14-membered, preferably 6- to 12-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocyclyl, or cycloalkyl ring, wherein the ring attached to the parental structure is the aryl ring; its non-limiting examples include:

, , and .

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5-membered or 6-membered. For example, its non-limiting examples include: imidazolyl, furyl, thienyl, thiazolyl, pyrazolyl, oxazolyl, pyrrolyl, tetrazolyl, pyridyl, pyrimidinyl, thiadiazole, pyrazine,

, , ,

etc.

The heteroaryl ring may be fused to an aryl, heterocyclyl, or cycloalkyl ring, wherein the ring attached to the parental structure is the heteroaryl ring; its non-limiting examples include:

, , , , ,

, , , and .

The term "alkoxy" refers to the group "alkyl-O-", wherein the alkyl is as defined above. Examples of alkoxy groups include methoxy, ethoxy, n-propoxy, isopropoxy, n-butoxy, tert-butoxy, sec-butoxy, n-pentoxy, n-hexoxy, and 1,2-dimethylbutoxy.

The term "haloalkyl" refers to an unbranched or branched alkyl group as defined above, wherein one or more hydrogen atoms are replaced by halogen. For example, when a residue is substituted with more than one halogen, it may be referred to by using a prefix corresponding to the number of halogen moieties attached. Dihaloalkyl and trihaloalkyl refer to alkyl groups substituted with two or three halogen groups, respectively, and the halogen groups may be, but are not necessarily, the same. Examples of haloalkyl groups include difluoromethyl ($-CHF_2$) and trifluoromethyl ($-CF_3$).

The term "haloalkoxy" refers to an alkoxy group as defined above, wherein one or more hydrogen atoms are replaced by halogen.

The term "hydroxyalkyl" refers to an alkyl group substituted with one or more hydroxy groups, wherein the alkyl is as defined above.

The term "hydroxy" refers to the -OH group.

The term "halogen" refers to fluorine, chlorine, bromine, or iodine.

The term "cyano" refers to -CN.

The term "nitro" refers to $-NO_2$.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

"Optionally" or "optional" means that the event or circumstance subsequently described may, but does not necessarily, occur, and this description includes instances where the event or circumstance occurs or does not occur. For example, "$C_{1-6}$ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

## DETAILED DESCRIPTION

The present disclosure is further described below with reference to examples; however, these examples are not intended to limit the scope of the present disclosure.

The structures of the compounds were determined by nuclear magnetic resonance (NMR) spectroscopy or/and mass spectrometry (MS). The NMR shifts (δ) are given in $10^{-6}$ (ppm). The NMR analyses were performed using a Bruker AVANCE-400 nuclear magnetic resonance instrument or Bruker AVANCE NEO 500M, with dimethyl sulfoxide-D6 (DMSO-$d_6$) and methanol-D4 ($CD_3OD$) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector), and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high performance liquid chromatographs.

The chiral HPLC analyses were performed using an Agilent 1260 DAD high performance liquid chromatograph.

The preparative high performance liquid chromatography was performed using Waters 2545-2767, Waters 2767-SQ Detector 2, Shimadzu LC-20AP, and Gilson GX-281 preparative chromatographs.

The preparative chiral chromatography was performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The silica gel column chromatography generally used Yantai Huanghai 200-300 mesh silica gel as the carrier.

The mean kinase inhibition rates and $IC_{50}$ values were determined using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, J&K, Accela ChemBio Inc., Shanghai Bide Pharmatech, and Chembee Chemicals.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen.

The hydrogen atmosphere means that the reaction flask was connected to a balloon containing about 1 L of hydrogen.

Hydrogenation reactions generally involved 3 cycles of vacuumization and hydrogen filling.

In the examples, the solutions were aqueous solutions unless otherwise specified.

In the examples, the reaction temperature was room temperature, i.e., 20 °C-30 °C, unless otherwise specified.

The reaction processes in the examples were monitored using thin-layer chromatography (TLC). The developing solvents used for reactions, the eluent systems of column chromatography used for compound purification, and the developing solvent systems of thin-layer chromatography included: A: a dichloromethane/methanol system, B: an n-hexane/ethyl acetate system, C: a petroleum ether/ethyl acetate system, and D: a petroleum ether/ethyl acetate/methanol system. The volume ratio of the solvents was adjusted based on the polarity of the compound, or by adding a small amount of basic or acidic reagents such as triethylamine and acetic acid.

The abbreviations used in the experiments below have the following meanings:

TFA: trifluoroacetic acid; DCM: dichloromethane; *m*-CPBA: 3-chloroperoxybenzoic acid; EtONa: sodium ethoxide; Boc: tert-butoxycarbonyl; MeOH: methanol; HBTU: *N,N,N',N'*-tetramethyl-O-(1H-benzotriazol-1-yl)uronium hexafluorophosphate; TsOH: p-toluenesulfonic acid; EtOAc: ethyl acetate; t-BuOH: tert-butyl alcohol; $PdCl_2(TPP)_2$: bis(triphenylphosphine)palladium(II) dichloride; XantPhos: 4,5-bis(diphenylphosphino)-9,9-dimethylxanthene; $Pd_2dba_3$: tris(dibenzylideneacetone)dipalladium(0); $PdCl_2(TPP)_2$: bis(triphenylphosphine)palladium(II) dichloride; XantPhos: 4,5-bis(diphenylphosphino)-9,9-dimethylxanthene; $Pd_2dba_3$: tris(dibenzylideneacetone)dipalladium(0); DMF: *N,N*-dimethylformamide; CDI: *N,N*-carbonyldiimidazole; ACN: acetonitrile; DMP: 1,1,1-tris(acetyloxy)-1,1-dihydro-1,2-benziodoxol-3(1H)-one (Dess-Martin periodinane); DMAP: 4-dimethylaminopyridine.

Example **1**

(*R*)-*N*-((*S*)-2-(Dimethylamino)-1-phenylethyl)-6-methyl-2-(((4-methylthiazol-2-yl)meth yl)amino)-5,8-dihydropyrido [3,4-*d*]pyrimidine-7(6H)-carboxamide

Step 1

Ethyl 4-(((R)-1-phenylethyl)amino)pentanoate (**1c**)

At room temperature, compound **1a** (50.0 g, 412.6 mmol) and compound **1b** (67.3 mL, 474.5 mmol) were dissolved in DCM (500 mL), and $NaBH(OAc)_3$ (174 g, 825 mmol) was slowly added in batches. The mixture was stirred overnight at room temperature. The reaction mixture was extracted with DCM/MeOH (9:1, 500 mL × 2). The organic phase was washed with saturated brine, dried over $Na_2SO_4$, and filtered, and the filtrate was concentrated under reduced pressure to give compound **1c** (crude, 80 g, 249 mmol). MS m/z (ESI): 250.3 $[M+H]^+$.

Step 2

Ethyl 4-((2-ethoxy-2-oxoethyl)((R)-1-phenylethyl)amino)pentanoate (**1e**)

At room temperature, compound **1c** (80.0 g, 249 mmol) and compound **1d** (31.7 mL, 320.8 mmol) were dissolved in DCM (1 L), and $NaBH(OAc)_3$ (203 g, 962 mmol) was slowly added in batches. The mixture was stirred at room temperature for 2 days. The reaction mixture was extracted with DCM/MeOH (9:1, 500 mL × 2). The organic phase was washed with saturated brine, dried over $Na_2SO_4$, and filtered, and the filtrate was concentrated under reduced pressure to give a crude product. The crude product was purified by silica gel flash column chromatography (eluent: 1-10% ethyl

acetate in petroleum ether) to give compound **1e** (40 g, 249 mmol).

MS m/z (ESI): 336.3 $[M+H]^+$.

Step 3

Ethyl

5-hydroxy-2-methyl-1-((R)-1-phenylethyl)-1,2,3,6-tetrahydropyridine-4-carboxylate (**1f**)

Compound **1e** (crude, 130.0 g) was dissolved in toluene (1.2 L), and potassium tert-butoxide (87.0 g, 776.8 mmol) was added in batches. The mixture was left to react at room temperature for 1.5 h. The reaction mixture was poured into a saturated ammonium chloride solution to adjust the pH to about 8, and the aqueous phase was isolated and extracted with dichloromethane (500 mL × 2). The organic phases were combined, washed with a saturated sodium chloride solution, dried over anhydrous sodium sulfate, filtered, and concentrated under reduced pressure to give a crude product. The crude product was purified by column chromatography (PE:EA = 30:1) to give compound **1f** (33.0 g, 119.9 mmol).

Step 4

(R)-6-Methyl-7-((R)-1-phenylethyl)-5,6,7,8-tetrahydropyrido[3,4-d]pyrimidine-2,4-diol **(1g)**

Compound **1f** (108.0 g, 392.7 mmol) was dissolved in methanol (1 L), and urea (89.7 g, 1492.8 mmol) and a solution of sodium methoxide in methanol (30%) (201.6 g, 1119.6 mmol) were added. The mixture was heated to 70 °C and left to react for 18 h. The reaction was stopped, and the reaction mixture was concentrated to dryness under reduced pressure. The pH of the reaction mixture was adjusted to about 8.0 with 3 M hydrochloric acid, and extraction was performed with DCM (1 L × 3). A large amount of white solid precipitated from the organic phase. The mixture was filtered, and the filter cake was compound **1g.** The filtrate was concentrated under reduced pressure, and the resulting residue was purified by column chromatography (DCM:MeOH = 10:1) to give compound **1g.** The products were combined to give compound **1g** (77.0 g, yield: 72.3%).

Step 5

(R)-6-Methyl-5,6,7,8-tetrahydropyrido[3,4-d]pyrimidine-2,4-diol **(1h)**

Compound **1g** (60.0 g, 210.4 mmol) was dissolved in methanol (900 mL), and Pd/C (12.0 g) was added. The system was purged with hydrogen three times. The mixture was left to react overnight at room temperature. The reaction mixture was filtered and concentrated under reduced pressure to give compound **1h** (crude, 43.0 g).

Step 6

(R)-2,4-Dichloro-6-methyl-5,6,7,8-tetrahydropyrido[3,4-d]pyrimidine (**1i**)

Compound **1h** (crude, 43.0 g) was suspended in phosphorus oxychloride (43 mL), and the suspension was heated to 100 °C and left to react overnight. The reaction mixture was concentrated under reduced pressure, and the resulting crude product was completely dissolved in iced water (300 mL). The solution was extracted with DCM (50 mL), and the aqueous phase was kept and directly used in the next step.

Step 7

tert-Butyl

(R)-2,4-dichloro-6-methyl-5,8-dihydropyrido[3,4-d]pyrimidine-7(6H)-carboxylate **(1j)**

The pH of the aqueous phase in step 6, which contained compound $_{1i}$, was adjusted to 8-9 with NEt**3**, and di-tert-butyl carbonate (77.7 g, 444.8 mmol) was added. The mixture was left to react at room temperature for 2 h. The reaction mixture was extracted with DCM (500 mL × 2), and the extract was dried over anhydrous sodium sulfate, filtered, and concentrated under reduced pressure. The resulting residue was purified by neutral alumina column chromatography (PE:EA = 10:1) to give compound **1j** (38.0 g, 119.6 mmol).

Step 8

tert-Butyl

(R)-2-chloro-6-methyl-5,8-dihydropyrido[3,4-d]pyrimidine-7(6H)-carboxylate **(1k)**

Compound **1j** (10.0 g, 31.5 mmol) was dissolved in ethanol (100 mL), and zinc powder (20.6 g) and glacial acetic acid (18.9 g, 314.7 mmol) were added. The mixture was heated to 80 °C and left to react for 1.5 h. The reaction mixture was filtered, and the pH of the filtrate was adjusted to 8-9 with $NEt_3$. The filtrate was concentrated under reduced pressure, and water (50 mL) was added to the resulting residue. The mixture was extracted with DCM (100 mL × 3), and the extract was concentrated under reduced pressure to give a crude product. The crude product was purified by reversed-phase column chromatography ($CH_3CN/H_2O$) to give compound **1k** (4.5 g, 15.9 mmol, yield: 50.4%).

Step 9

tert-Butyl

(R)-6-methyl-2-(((4-methylthiazol-2-yl)methyl)amino)-5,8-dihydropyrido[3,4-d]pyrimi dine-7(6H)-carboxylate **(1m)**

Compound **1l** (50 mg, 0.39 mmol) was dissolved in 1,4-dioxane (5 mL), and subsequently, compound **1k** (115 mg, 0.4 mmol), $Cs_2CO_3$ (390 mg, 1.2 mmol), and dichloro[1,3-bis(2,6-di-3-pentylphenyl)imidazol-2-ylidene](3-chloropyridyl) palladium(I I) (31 mg, 0.04 mmol) were sequentially added. The mixture was stirred at 110 °C for 16 h. The filtrate was concentrated under reduced pressure, and the resulting residue was purified by column chromatography (PE:EA = 5:1) to give compound **1m** (80 mg, 0.21 mmol, yield: 53%).

MS m/z (ESI): 376.5.

Step 10

(R)-6-Methyl-N-((4-methylthiazol-2-yl)methyl)-5,6,7,8-tetrahydropyrido[3,4-d]pyrimid in-2-amine **(1n)**

Compound **1m** (80 mg, 0.21 mmol) was added to a 50 mL flask, and 4 mL of a mixed solvent (DCM:TFA = 2:1) was subsequently added. After the reaction was complete, the reaction mixture was concentrated under reduced pressure to give **1n** (80 mg, crude). MS m/z (ESI): 276.4.

Step 11

(*R*)-*N*-((*S*)-2-(Dimethylamino)-1-phenylethyl)-6-methyl-2-(((4-methylthiazol-2-yl)meth yl)amino)-5,8-dihydropyrido [3,4-*d*]pyrimidine-7(6H)-carboxamide **(1)**

Compound **1o** (54 mg, 0.32 mmol) was dissolved in a solution of DMF (2.0 mL), and CDI (104 mg, 0.64 mmol) and $NEt_3$ (63 mg, 0.63 mmol) were subsequently added. The mixture was stirred at room temperature for 0.1 h. **1n** (80 mg, crude) was then added, and the mixture was stirred overnight at room temperature. The reaction mixture was extracted with ethyl acetate (10 mL × 3). The organic phase was dried over $Na_2SO_4$ and filtered, and the filtrate was concentrated under reduced pressure to give a crude product. The crude product was purified by HPLC to give compound 1 (6.6 mg, yield: 15%).

MS m/z (ESI): 466.8.

$^1H$ NMR (400 MHz, $CD_3OD$) δ 8.13 (s, 1H), 7.42-7.29 (m, 5H), 6.97 (d, *J* = 1.2 Hz, 1H), 5.36-5.31 (m, 1H), 4.86-4.73 (m, 4H), 4.16 (d, *J* = 18.4 Hz, 1H), 3.49-3.40 (m, 1H), 3.27-3.20 (m, 1H), 2.92-2.80 (m, 7H), 2.55 (d, J = 15.6, 1H), 2.38 (s, 3H), 1.05 (d, *J* = 6.8 Hz, 3H).

Example 2

(*R*)-*N*-((*S*)-2-(Dimethylamino)-1-phenylethyl)-6-methyl-2-(((*S*)-1-(4-methylthiazol-2-yl )ethyl)amino)-5,8-dihydropyrido [3,4-*d*]pyrimidine-7(6H)-carboxamide

2-1

($R$)-$N$-(($S$)-2-(Dimethylamino)-1-phenylethyl)-6-methyl-2-((($R$)-1-(4-methylthiazol-2-yl )ethyl)amino)-5,8-dihydropyrido [3,4-$d$]pyrimidine-7(6H)-carboxamide

2-2

Boc Boc H NH2 H2N 1o 1k 2a 2b 2c 2 SFC + 2-1 2-2

With reference to the preparation method for compound **1,** compound **2a** (125 mg, 0.88 mmol) was subjected to three steps to give compound **2** (85 mg, 0.19 mmol).

MS m/z (ESI): 480.2.

Compound **2** was resolved by chiral column chromatography (column: ChiralPak IG-3 100 × 4.6 mm I.D., 3 μm; mobile phase: A: 50% supercritical $CO_2$ fluid, B: 50% ethanol (0.05% DEA)) to give compounds **2-1** and **2-2.**

Compound **2-1** (retention time: 3.364 min)

MS m/z (ESI): 480.2.

$^1$H NMR (400 MHz, $CD_3OD$) δ 8.10 (s, 1H), 7.39-7.17 (m, 5H), 6.92 (d, J = 1.3 Hz, 1H), 5.40 (q, J = 7.0 Hz, 1H), 5.04 (dd, J = 10.8, 4.3 Hz, 1H), 4.83-4.59 (m, 2H), 4.13 (d, J = 18.4 Hz, 1H), 2.95-2.78 (m, 2H), 2.58-2.41 (m, 2H), 2.38 (d, J = 1.0 Hz, 3H), 2.34 (s, 6H), 1.63 (d, J = 7.0 Hz, 3H), 1.03 (d, J = 6.7 Hz, 3H).

Compound **2-2** (retention time: 5.417 min)

MS m/z (ESI): 480.2.

$^1$H NMR (400 MHz, $CD_3OD$) δ 8.09 (s, 1H), 7.44-7.18 (m, 5H), 6.93 (d, $J$ = 1.2 Hz, 1H), 5.39 (q, $J$ = 7.0 Hz, 1H), 5.12 (dd, $J$ = 11.0, 4.2 Hz, 1H), 4.81-4.67 (m, 2H), 4.10 (d, $J$ = 18.3 Hz, 1H), 3.04 (dd, $J$ = 12.9, 11.0 Hz, 1H), 2.88 (dd, $J$ = 15.6, 5.7 Hz, 1H), 2.67 (dd, $J$ = 13.0, 4.3 Hz, 1H), 2.54 (s, 1H), 2.48 (s, 6H), 2.39 (s, 3H), 1.63 (d, $J$ = 7.0 Hz, 3H), 1.02 (d, $J$ = 6.8 Hz, 3H).

Example **3**

(*R*)-*N*-((*S*)-2-(Bis(methyl-d3)amino)-1-phenylethyl)-6-methyl-2-(((*S*)-1-(4-methylthiazo 1-2-yl)ethyl)amino)-5,8-dihydropyrido[3,4-*d*]pyrimidine-7(6H)-carboxamide

**3-1**

(*R*)-*N*-((*S*)-2-(Bis(methyl-d3)amino)-1-phenylethyl)-6-methyl-2-(((*R*)-1-(4-methylthiazo 1-2-yl)ethyl)amino)-5,8-dihydropyrido[3,4-*d*]pyrimidine-7(6H)-carboxamide

**3-2**

**3a** **3b** **3c** **3d** **3e**

SFC

**3** **3-1** **3-2**

Step 1

Benzyl (S)-(2-(bis(methyl-d3)amino)-2-oxo-1-phenylethyl)carbamate (**3c**)

At room temperature, compound **3a** (500 mg, 1.753 mmol) and HBTU (1329 mg, 3.5 mmol) were dissolved in DCM (7 mL), and the solution was stirred for 5 min. $K_2CO_3$ (726 mg, 5.26 mmol) and compound **3b** (134.4 mg, 2.63 mmol) were added. The reaction was stirred at room temperature for 12 h. The reaction mixture was extracted, and the extract was concentrated and dried to give a crude product. The crude product was separated and purified by column

chromatography to give compound **3c** (900 mg, 2.83 mmol).
MS m/z (ESI): 319.2 $[M+H]^+$.

Step 2

(S)-2-Amino-N,N-bis(methyl-d3)-2-phenylacetamide **(3d)**

In a hydrogen atmosphere, 10% palladium on carbon (Pd/C) was added to a solution of compound **3c** (900 mg, 2.83 mmol) in ethyl acetate (15 mL). The mixture was stirred at room temperature for 2 h. The reaction mixture was filtered and concentrated to give **3d** (crude, 260 mg). The crude product (260 mg) was used directly in the next step.
MS m/z (ESI): 185.1 $[M+H]^+$.

Step 3

(S)-N1,N1-Bis(methyl-d3)-2-phenylethane-1,2-diamine **(3e)**

In a nitrogen atmosphere, $LiAlH_4$ (82.0 mg, 2.17 mmol) was added to anhydrous THF (5.0 mL). After the mixture was cooled to -78 °C, a solution of compound **3d** in THF was added dropwise, and the mixture was stirred for 30 min. Subsequently, the reaction mixture was heated to 50 °C, left to react for 2 h, and quenched with an ammonium chloride solution. After extraction, concentration, and drying, a crude product was obtained. The crude product was separated and purified by preparative HPLC to give compound **3e** (40.0 mg, yield: 21%).
MS m/z (ESI): 171.1 $[M+H]^+$.

Step 4

(*R*)-*N*-((*S*)-2-(Bis(methyl-d3)amino)-1-phenylethyl)-6-methyl-2-((1-(4-methylthiazol-2-yl)ethyl)amino)-5,8-dihydropyrido [3,4-*d*]pyrimidine-7(6H)-carboxamide **(3)**

Compound **3** (52 mg, 0.11 mmol) was synthesized by referring to the preparation method for compound **1** and replacing compound **1a** with compound **3e.**
MS m/z (ESI): 486.3.

Step 5

Preparation of compounds **3-1** and **3-2**

Compound **3** was resolved by chiral column chromatography (column: ChiralPak IG-3 100 × 4.6 mm I.D., 3 μm; mobile phase: A: 50% supercritical $CO_2$ fluid, B: 50% ethanol (0.05% DEA)) to give compounds **3-1** and **3-2.**
Compound **3-1** (retention time: 3.288 min)
MS m/z (ESI): 486.2.
$^1H$ NMR (400 MHz, $CD_3OD$) δ 8.10 (s, 1H), 7.47-7.11 (m, 5H), 6.92 (d, *J* = 1.1 Hz, 1H), 5.40 (q, *J* = 7.0 Hz, 1H), 5.04 (dd, *J* = 10.8, 4.3 Hz, 1H), 4.82-4.63 (m, 2H), 4.13 (d, *J* = 18.4 Hz, 1H), 2.99-2.80 (m, 2H), 2.62-2.45 (m, 2H), 2.38 (s, 3H), 1.63 (d, *J* = 7.0 Hz, 3H), 1.03 (d, *J* = 6.7 Hz, 3H).
Compound **3-2** (retention time: 5.344 min)
MS m/z (ESI): 486.2.
$^1H$ NMR (400 MHz, $CD_3OD$) δ 8.09 (s, 1H), 7.46-7.20 (m, 5H), 6.93 (d, *J* = 1.1 Hz, 1H), 5.45-5.33 (m, 1H), 5.06 (dd, *J* = 10.8, 4.3 Hz, 1H), 4.82-4.70 (m, 2H), 4.09 (d, *J* = 18.4 Hz, 1H), 2.98-2.84 (m, 2H), 2.60-2.46 (m, 2H), 2.39 (s, 3H), 1.63 (d, *J* = 7.0 Hz, 3H), 1.02 (d, *J* = 6.7 Hz, 3H).

Example **4**

(*R*)-2-(((*S*)-1-(4-Cyclopropylthiazol-2-yl)ethyl)amino)-*N*-((*S*)-2-(dimethylamino)-1-phe nylethyl)-6-methyl-5,8-dihydropyrido[3,4-*d*]pyrimidine-7(6H)-carboxamide

Step 1

(S,E)-N-((4-Bromothiazol-2-yl)methylene)-2-methylpropane-2-sulfinamide **(4c)**

Compound **4a** (3.0 g, 15.6 mmol) was dissolved in DCM (30 mL), and compound **4b** (1.89 g, 15.6 mmol) and $CuSO_4$ (4.99 g, 31.2 mmol) were added. The reaction mixture was stirred at room temperature for 16 h in a $N_2$ atmosphere. The reaction mixture was filtered and concentrated under reduced pressure to give compound **4c** (crude, 4.5 g). The crude product was directly used in the next step.

MS m/z (ESI): 295.0 $[M+H]^+$.

Step 2

(S)-N-((S)-1-(4-Bromothiazol-2-yl)ethyl)-2-methylpropane-2-sulfinamide **(4d)**

In a $N_2$ atmosphere, at -50 °C, a solution of methylmagnesium bromide in THF (3.0 M, 15.2 mL, 45.7 mmol) was added to a solution of compound **4c** (4.5 g, crude) in DCM (50 mL). The mixture was stirred at -50 °C for 4 h, then warmed to room temperature, and stirred for 18 h. The reaction mixture was quenched with 20 mL of a saturated $NH_4Cl$ solution, water (300 mL) was added, and extraction was performed with DCM (100 mL × 3). The organic phase was dried over $Na_2SO_4$ and filtered, and the filtrate was concentrated under reduced pressure to give a crude product. The crude product was purified by silica gel flash column chromatography (eluent: 0-60% ethyl acetate in petroleum ether) to give compound **4d** (2.5 g, 8.0 mmol, yield: 52.7%).

MS m/z (ESI): 311.0 $[M+H]^+$.

Step 3

(S)-N-((S)-1-(4-Cyclopropylthiazol-2-yl)ethyl)-2-methylpropane-2-sulfinamide **(4f)**

Compound **4e** (475.3 mg, 3.2 mmol), Pd(OAc)**2** (36.1 mg, 0.16 mmol), CataCXium A (86.39 mg, 0.241 mmol), and $Cs_2CO_3$ (1570.1 mg, 4.82 mmol) were added to a mixed solution of compound **4d** (500 mg, 1.6 mmol) in toluene (5.0 mL) and water (0.5 mL). In a $N_2$ atmosphere, the reaction mixture was heated to 100 °C and stirred for 16 h. Water (300 mL) was added to the reaction mixture, and extraction was performed with EtOAc (100 mL × 3). The organic phase was dried over $Na_2SO_4$ and filtered, and the filtrate was concentrated under reduced pressure to give a crude product. The crude product was purified by silica gel flash column chromatography (eluent: 0-60% MeOH in DCM) to give compound **4f** (330 mg, 1.21 mmol, yield: 75.4%).

MS m/z (ESI): $[M+H]^+$.

$^1$H NMR (400 MHz, DMSO-$d_6$) δ 7.14 (s, 1H), 6.14 (d, *J* = 7.1 Hz, 1H), 4.63-4.54 (m, 1H), 2.05-1.99 (m, 1H), 1.50 (d, *J* = 6.8 Hz, 3H), 1.13 (s, 9H), 0.90-0.83 (m, 2H), 0.85-0.74 (m, 2H).

Step 4

(S)-1-(4-Cyclopropylthiazol-2-yl)ethan-1-amine **(4g)**

At room temperature, HCl (4.0 M in dioxane, 3.028 mL, 12.113 mmol) was added to a solution of compound **4f** (330 mg, 1.21 mmol) in methanol. After 1 h of stirring, the reaction mixture was concentrated under reduced pressure. Acetonitrile (10 mL) and water (10 mL) were added to the residue, and the mixture was concentrated under reduced pressure to remove acetonitrile and lyophilized to give compound **4g** (280 mg, 1.16 mmol, yield: 95.8%).

MS m/z (ESI): 169.1 [M+H]$^+$.

$^1$H NMR (400 MHz, DMSO-$d_6$) δ 8.77 (s, 3H), 7.33 (s, 1H), 4.74-4.68 (m, 1H), 2.11-2.07 (m, 1H), 1.58 (d, J = 6.8 Hz, 3H), 0.93-0.88 (m, 2H), 0.84-0.80 (m, 2H).

With reference to the synthesis method for compound **1,** compound **4g** was subjected to three steps to synthesize compound **4** (7.7 mg, 0.015 mmol).

MS m/z (ESI): 506.9.

$^1$H NMR (400 MHz, $CD_3OD$) δ 8.10 (s, 1H), 7.52-7.14 (m, 5H), 6.84 (s, 1H), 5.39-5.34 (m, 1H), 5.21 (dd, *J* = 11.2, 4.1 Hz, 1H), 4.84-4.64 (m, 2H), 4.14 (d, *J* = 18.3 Hz, 1H), 3.26-3.06 (m, 1H), 2.92-2.83 (m, 2H), 2.62 (s, 6H), 2.52 (d, *J* = 15.6 Hz, 1H), 2.06-1.99 (m, 1H), 1.61 (d, *J* = 7.0 Hz, 3H), 1.03 (d, *J* = 6.8 Hz, 3H), 0.96-0.89 (m, 2H), 0.87-0.72 (m, 2H).

Example **5**

(*R*)-2-(((*R*)-1-(4-Cyclopropylthiazol-2-yl)ethyl)amino)-*N*-((*S*)-2-(dimethylamino)-1-phe nylethyl)-6-methyl-5,8-dihydropyrido[3,4-*d*]pyrimidine-7(6H)-carboxamide

**5**

With reference to the synthesis method for compound **4,** starting material **5a** (2 g, 10.4 mmol) was subjected to 7 steps to prepare compound **5** (11.6 mg, 0.023 mmol).

MS m/z (ESI): 506.5.

$^1$H NMR (400 MHz, $CD_3OD$) δ 8.10 (d, *J* = 2.0 Hz, 1H), 7.52-7.25 (m, 5H), 6.85 (d, *J* = 1.3 Hz, 1H), 5.45-5.26 (m, 2H), 4.85-4.71 (m, 2H), 4.11 (d, *J* = 18.1 Hz, 1H), 3.77-3.50 (m, 1H), 3.39-3.35 (m, 1H), 2.94 (s, 6H), 2.88 (dd, *J* = 16.3, 5.4 Hz, 1H), 2.53 (d, *J* = 15.5 Hz, 1H), 2.10-1.95 (m, 1H), 1.61 (d, *J* = 7.0 Hz, 3H), 1.04 (d, *J* = 6.6 Hz, 3H), 0.93-0.89 (m, 2H),

0.86-0.74 (m, 2H).

Example **6**

(*R*)-2-(((4-Cyclopropylthiazol-2-yl)methyl)amino)-*N*-((*S*)-2-(dimethylamino)-1-phenyle thyl)-6-methyl-5,8-dihydropyrido [3,4-*d*]pyrimidine-7(6H)-carboxamide

**6**

Compound **6** (1.1 mg, 0.002 mmol) was synthesized with reference to the preparation method for compound **1.**
MS m/z (ESI): 492.5.
$^1$H NMR (400 MHz, $CD_3OD$) δ 8.13 (s, 1H), 7.48-7.20 (m, 5H), 6.89 (s, 1H), 5.31 (dd, *J* = 11.4, 4.2 Hz, 1H), 4.82-4.66 (m, 4H), 4.16 (d, *J* = 18.3 Hz, 1H), 3.43-3.37 (m, 1H), 3.18 (dd, *J* = 13.1, 4.1 Hz, 1H), 2.89 (dd, *J* = 15.7, 5.6 Hz, 1H), 2.81 (s, 6H), 2.55 (d, *J* = 15.6Hz, 1H), 2.06-2.00 (m, 1H), 1.05 (d, *J* = 6.8 Hz, 3H), 0.96-0.86 (m, 2H), 0.86-0.72 (m, 2H).

Example **7**

(*R*)-2-(((4-Cyclopropylthiazol-2-yl)methyl)amino)-*N*-((*S*)-2-(bis(methyl-d3)amino)-1-p henylethyl)-6-methyl-5,8-dihydropyrido[3,4-*d*]pyrimidine-7(6H)-carboxamide

**7**

Compound **7** (5.7 mg, 0.012 mmol) was synthesized by referring to the preparation method for compound 1 and replacing compound **1a** with compound **3e.**
MS m/z (ESI): 472.9.
$^1$H NMR (400 MHz, $CD_3OD$) δ 8.12 (d, *J* = 4.7 Hz, 1H), 7.45-7.26 (m, 5H), 6.97-6.89 (m, 1H), 5.36 (dd, *J* = 11.7, 4.1 Hz, 1H), 4.84-4.72 (m, 4H), 4.15 (dd, *J* = 18.5, 4.2 Hz, 1H), 3.54-3.42 (m, 1H), 3.36-3.17 (m, 2H), 2.96-2.73 (m, 1H), 2.53 (dt, *J* = 15.7, 3.1 Hz, 1H), 2.38 (s, 3H), 1.04 (dd, *J* = 7.1, 2.9 Hz, 3H).

Example **8**

(*R*)-2-(((4-Cyclopropylthiazol-2-yl)methyl)amino)-*N*-((*S*)-2-(bis(methyl-d3)amino)-1-p henylethyl)-6-methyl-5,8-dihydropyrido[3,4-*d*]pyrimidine-7(6H)-carboxamide

8

Compound **8** (2.3 mg, 0.005 mmol) was synthesized by referring to the preparation method for compound **1** and replacing compound **1a** with compound **3e.**

MS m/z (ESI): 498.5.

$^1$H NMR (400 MHz, $CD_3OD$) δ 8.13 (s, 1H), 7.49-7.19 (m, 5H), 6.90 (s, 1H), 5.33-5.26 (m, 1H), 4.82-4.73 (m, 4H), 4.16 (d, *J* = 18.3 Hz, 1H), 3.84-3.31 (m, 1H), 3.20-3.07 (m, 1H), 2.89 (dd, *J* = 15.6, 5.6 Hz, 1H), 2.55 (d, *J* = 15.6 Hz, 1H), 2.07-1.99 (m, 1H), 1.05 (d, *J* = 6.8 Hz, 3H), 0.96-0.88 (m, 2H), 0.84-0.77 (m, 2H).

Example **9**

(*R*)-2-(((*S*)-1-(4-Cyclopropylthiazol-2-yl)ethyl)amino)-*N*-((*S*)-2-(bis(methyl-d3)amino)-1-phenylethyl)-6-methyl-5,8-dihydropyrido[3,4-*d*]pyrimidine-7(6H)-carboxamide

9

Compound **9** (11.3 mg, 0.022 mmol) was synthesized by referring to the preparation method for compound **4** and replacing compound **1a** with compound **3e.**

MS m/z (ESI): 512.5.

$^1$H NMR (400 MHz, $CD_3OD$) δ 8.11 (s, 1H), 7.43-7.24 (m, 5H), 6.84 (s, 1H), 5.39-5.35 (m, 1H), 5.28 (dd, *J* = 11.5, 4.1 Hz, 1H), 4.82-4.64 (m, 2H), 4.15 (d, *J* = 18.2 Hz, 1H), 3.38-3.33 (m, 1H), 3.13-3.09 (m, 1H), 2.88 (dd, *J* = 15.6, 5.6 Hz, 1H), 2.55 (d, *J* = 15.6 Hz, 1H), 2.06-1.99 (m, 1H), 1.61 (d, *J* = 7.0 Hz, 3H), 1.04 (d, *J* = 6.8 Hz, 3H), 0.95-0.89 (m, 2H), 0.84-0.77 (m, 2H).

Example **10**

(*R*)-2-(((*R*)-1-(4-Cyclopropylthiazol-2-yl)ethyl)amino)-*N*-((*S*)-2-(bis(methyl-d3)amino)-1-phenylethyl)-6-methyl-5,8-dihydropyrido[3,4-*d*]pyrimidine-7(6H)-carboxamide

10

Compound **10** (1.4 mg, 0.003 mmol) was synthesized by referring to the preparation method for compound **4** and replacing compound **1a** with compound **3e.**

MS m/z (ESI): 512.9.

$^1$H NMR (400 MHz, $CD_3OD$) δ 8.10 (s, 1H), 7.52-7.21 (m, 5H), 6.85 (s, 1H), 5.46-5.21 (m, 2H), 4.81-4.66 (m, 2H), 4.11 (d, *J* = 18.2 Hz, 1H), 3.45-3.37 (m, 1H), 3.23-3.17 (dd, *J* = 13.0, 4.2 Hz, 1H), 2.88 (dd, *J* = 15.6, 5.6 Hz, 1H), 2.53 (d, *J* = 15.6 Hz, 1H), 2.08-1.99 (m, 1H), 1.61 (d, *J*= 7.0 Hz, 3H), 1.03 (d, *J* = 6.8 Hz, 3H), 0.96-0.85 (m, 2H), 0.85-0.76 (m, 2H).

Example **11**

(*R*)-*N*-((*S*)-2-(Dimethylamino)-1-phenylethyl)-2-(((S)-1-(4-(hydroxymethyl)thiazol-2-yl )ethyl)amino)-6-methyl-5,8-dihydropyrido[3,4-d]pyrimidine-7(6H)-carboxamide

11-1

(*R*)-*N*-((*S*)-2-(Dimethylamino)-1-phenylethyl)-2-(((R)-1-(4-(hydroxymethyl)thiazol-2-yl )ethyl)amino)-6-methyl-5,8-dihydropyrido[3,4-d]pyrimidine-7(6H)-carboxamide

11-2

Compound **11** (19 mg, 0.038 mmol) was synthesized with reference to the preparation method for compound **2.**

Compound **11** was resolved by chiral column chromatography (column: ChiralPak IG 250 × 30 mm I.D., 5 μm; mobile phase: A: 55% supercritical $CO_2$ fluid, B: 45% methanol [0.1% $NH_3$ (7 M)]) to give compounds **11-1** and **11-2.**

Compound **11-1** (retention time: 4.848 min)

MS m/z (ESI): 496.2.

$^1$H NMR (400 MHz, DMSO-$d_6$) δ 8.12 (s, 1H), 7.74 (d, J = 7.9 Hz, 1H), 7.35-7.26 (m, 4H), 7.19 (s, 2H), 6.78 (d, J = 7.6 Hz, 1H), 5.34-5.29 (m, 1H), 5.25 (t, J = 5.8 Hz, 1H), 4.87 (q, J = 7.8 Hz, 1H), 4.69-4.59 (m, 2H), 4.51 (dd, J = 5.7, 1.1 Hz, 2H), 3.93 (d, J = 18.7 Hz, 1H), 2.77 (dd, J = 15.6, 5.6 Hz, 1H), 2.62 (dd, J = 12.3, 9.4 Hz, 1H), 2.43 (d, J = 15.3 Hz, 1H), 2.34

(dd, J = 12.4, 5.8 Hz, 1H), 2.17 (s, 6H), 1.55 (d, J = 7.0 Hz, 3H), 0.94 (d, J = 6.6 Hz, 3H).

Compound **2-2** (retention time: 5.503 min)

MS m/z (ESI): 496.2.

$^1$H NMR (400 MHz, DMSO-$d_6$) δ 8.13 (s, 1H), 7.74 (d, J = 7.9 Hz, 1H), 7.35-7.25 (m, 4H), 7.22-7.16 (m, 2H), 6.79 (d, J = 7.7 Hz, 1H), 5.34-5.29 (m, 1H), 5.25 (t, J = 5.7 Hz, 1H), 4.86 (q, J = 7.7 Hz, 1H), 4.68-4.49 (m, 4H), 3.96 (d, J = 18.6 Hz, 1H), 2.77 (dd, J = 15.6, 5.6 Hz, 1H), 2.65-2.58 (m, 1H), 2.36-2.30 (m, 1H), 2.15 (s, 6H), 2.00 (q, J = 7.0, 6.5 Hz, 1H), 1.55 (d, J = 7.0 Hz, 3H), 0.94 (d, J = 6.7 Hz, 3H).

## Biological Evaluations

The present disclosure is further described and explained below with reference to test examples. However, these examples are not intended to limit the scope of the present disclosure.

### Test Example 1

Test Example 1. Assay for Inhibitory Activity of Compounds of Present Disclosure Against Ovarian Cancer Cells (OVCAR3)

1.1. Experimental materials and instruments (see Table 1)

Table 1. Experimental materials and instruments

| Instrument name | Equipment manufacturer | Model |
|---|---|---|
| RPMI 1640 culture medium | Gibco | A1049101 |
| FBS | Gibco | 11965118 |
| CellTiter-Glo® kit | Promega | G7572 |
| 96-well white culture plate | Corning | 3610 |
| Envision | PerkinElmer | 2015 |
| Plate shaker | Beijing Jiayuan | MB-100-2A |

1.2. Experimental procedure

Ovarian cancer cells OVCAR3 (source: Nanjing Cobioer Biosciences Co., Ltd.) were cultured in an RPMI 1640 culture medium supplemented with 10% FBS in a cell incubator at 37 °C with 5% $CO_2$. On day one, the cells were plated in a 96-well plate at a cell concentration of 2500 cells/well and cultured overnight in an incubator. On day two, the cells were treated with compounds. The highest compound concentration was 10 μM and was diluted 3-fold to obtain 9 concentrations, and the final concentration of DMSO was 0.1%. After the cells were cultured in the incubator for another 5 days, the cell viability was measured using a Celltiter Glo assay kit (Promega) by a method consistent with that provided by the kit. Data were processed using GraphPad Prism 8, and $IC_{50}$ was calculated.

Calculation formula: Y = Bottom + (Top - Bottom)/(1 + 10^((LogIC50 - X) × HillSlope)).

X: the logarithm of the compound concentration; Y: % inhibition; Bottom: bottom; Top: top; HillSlope: Hill slope.

Table 2. The $IC_{50}$ (nM) of the compounds of the present disclosure against OVCAR3

| Compound No. | $IC_{50}$ (nM) |
|---|---|
| **1** | 10.15 |
| **2** | 3.98 |
| **2-1** | <1.5 |
| **2-2** | 66.04 |
| **3-1** | <1.5 |

(continued)

| Compound No. | $IC_{50}$ (nM) |
|---|---|
| **3-2** | 101.41 |
| **4** | <1.5 |
| **5** | 17.51 |
| **6** | 10.71 |
| **7** | 11.24 |
| **8** | 9.89 |
| **9** | <1.5 |
| **10** | 19.27 |
| **11-1** | 1.89 |
| **11-2** | 190 |
| **Janssen-01** | 22.22 |

The inhibitory activity of the compounds of Examples **2-1, 3-1, 4, 9, and 11-1** of the present disclosure against OVCAR3 tumor cells was significantly better than that of the disclosed compound **Janssen-01.**

**Janssen-01**

was prepared with reference to the method provided in WO2022064009A.

**Test Example 2**

Test Example 2. Assay for Inhibitory Activity of Compounds of Present Disclosure Against CDKs

2.1. Experimental procedure

An ADP-Glo kinase assay was used for testing the activity of CDKs. The compounds were diluted in 384-well plates using Echo. The starting concentration was 10 μM and was diluted 3-fold to obtain 10 concentration points, and each concentration was tested in duplicate. The final concentration of DMSO in the assay system was 1%. CDK solutions (final concentrations: 16.5 nM CDK1/CyclinB, 1 nM CDK2/CyclinE1, 16.3 nM CDK4/CyclinD1, 15.7 nM CDK6/CyclinD3, 80 nM CDK7/Cyclin H/MAT1, and 15.3 nM CDK9/Cyclin T1) prepared with assay buffer were added at 2.5 μL, and the enzymes and compounds were pre-incubated at room temperature for 10 min. ATP (concentration: $K_m$) & substrate solutions (20 μM ATP & 0.1 mg/mL histone H1 for CDK1/CyclinB, 15 μM ATP & 0.1 mg/mL histone H1 for CDK2/CyclinE1, 200 μM ATP & 0.2 mg/mL DYRKtide for CDK4/CyclinD1, 200 μM ATP & 0.1 mg/mL histone H1 for CDK6/CyclinD3, 70 μM ATP & 0.2 mg/mL MBP for CDK7/Cyclin H/MAT1, and 60 μM ATP & 0.2 mg/mL PDKtide for CDK9/Cyclin T1) prepared with assay buffer were added at 2.5 μL. After thorough mixing, the plates were incubated at room temperature (for 120 min for CDK1/4/9 and for 60 min for CDK2/6/7). 4 μL of the ADP-Glo reagent was added, and the plates were incubated at room temperature for 40 min. 8 μL of the kinase assay reagent was added, and the plates were incubated at room temperature for 40 min. Readings were taken using an Envision multimode microplate reader. Data were processed using GraphPad Prism 8, and $IC_{50}$ was calculated.

Calculation formula: $Y = Bottom + (Top - Bottom)/(1 + 10^{((LogIC50 - X) \times HillSlope)})$.

X: the logarithm of the compound concentration; Y: % inhibition; Bottom: bottom; Top: top; HillSlope: Hill slope.

Table 3. The inhibitory activity ($IC_{50}$, nM) of the compounds of the present disclosure against CDKs

| Example | CDK1 | CDK2 | CDK4 | CDK 6 | CDK7 | CDK9 |
|---|---|---|---|---|---|---|
| **2-1** | >1000 | 711 | >1000 | >1000 | 0.96 | >1000 |
| **4** | >1000 | 571 | >1000 | >1000 | 0.55 | >1000 |

**Claims**

**1.** A compound represented by formula (I') or a pharmaceutically acceptable salt thereof:

(I') ,

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;

m is selected from the group consisting of 0, 1, 2, 3, and 4;

$L_1$ is selected from the group consisting of a linking bond and $C_{1-3}$ alkylene, wherein the $C_{1-3}$ alkylene is optionally substituted with one or more $R^B$, and $R^B$ is selected from the group consisting of deuterium, halogen, hydroxy, oxo, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, hydroxy, halogen, $C_{1-6}$ alkyl, $C_{1-6}$ haloalkyl, and deuterated $C_{1-6}$ alkyl;

$R^2$, $R^3$, $R^4$, $R^5$, and $R^6$ are each independently selected from the group consisting of hydrogen and deuterium;

$R^7$ is $C_{1-3}$ alkyl, and the $C_{1-3}$ alkyl is optionally substituted with one or more deuterium atoms;

$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;

ring A is selected from the group consisting of phenyl and 5- to 6-membered heteroaryl;

$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally

substituted with one or more deuterium atoms or halogens;
n is selected from the group consisting of 0, 1, 2, 3, and 4;
$R^9$ is hydrogen or deuterium;
$L_3$ is $C_{1-3}$ alkylene, wherein the $C_{1-3}$ alkylene is optionally substituted with one or more $R^E$, and $R^E$ is deuterium;
$R^{10}$ and $R^{11}$ are each independently $C_{1-3}$ alkyl, wherein the $C_{1-3}$ alkyl is optionally substituted with one or more $R^F$, and $R^F$ is deuterium.

**2.** The compound or the pharmaceutically acceptable salt thereof according to claim 1, being a compound represented by formula (I) or a pharmaceutically acceptable salt thereof:

(I) ,

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;
m is selected from the group consisting of 0, 1, 2, 3, and 4;
$L_1$ is selected from the group consisting of a linking bond and $C_{1-3}$ alkylene, wherein the $C_{1-3}$ alkylene is optionally substituted with one or more $R^B$, and $R^B$ is selected from the group consisting of deuterium, halogen, hydroxy, oxo, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, halogen, $C_{1-6}$ alkyl, $C_{1-6}$ haloalkyl, and deuterated $C_{1-6}$ alkyl;
$R^2$, $R^3$, $R^4$, $R^5$, and $R^6$ are each independently selected from the group consisting of hydrogen and deuterium;
$R^7$ is $C_{1-3}$ alkyl, and the $C_{1-3}$ alkyl is optionally substituted with one or more deuterium atoms;
$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;
ring A is selected from the group consisting of phenyl and 5- to 6-membered heteroaryl;
$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;
n is selected from the group consisting of 0, 1, 2, 3, and 4;
$R^9$ is hydrogen or deuterium;
$L_3$ is $C_{1-3}$ alkylene, wherein the $C_{1-3}$ alkylene is optionally substituted with one or more $R^E$, and $R^E$ is deuterium;

$R^{10}$ and $R^{11}$ are each independently $C_{1-3}$ alkyl, wherein the $C_{1-3}$ alkyl is optionally substituted with one or more $R^F$, and $R^F$ is deuterium.

**3.** The compound or the pharmaceutically acceptable salt thereof according to claim 1, being a compound represented by formula (I'-A) or a pharmaceutically acceptable salt thereof:

(I'-A) ,

wherein:

$R^1$ is selected from the group consisting of deuterium, cyano, $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, -(C=O)NH-$C_{1-6}$ alkyl, and -(C=O)$NH_2$, wherein the $C_{1-6}$ alkyl, $C_{2-6}$ alkynyl, $C_{1-6}$ alkoxy, -S-$C_{1-6}$ alkyl, $C_{1-6}$ alkylene-3- to 6-membered cycloalkyl, -$C_{1-6}$ alkylene-3- to 6-membered heterocycloalkyl, 3- to 6-membered cycloalkyl, 3- to 6-membered heterocycloalkyl, phenyl, 5- to 6-membered heteroaryl, -NH(C=O)-O$C_{1-6}$ alkyl, and -(C=O)NH-$C_{1-6}$ alkyl are each independently and optionally substituted with one or more $R^A$, and $R^A$ is selected from the group consisting of deuterium, halogen, hydroxy, amino, oxo, $C_{1-6}$ alkyl, $C_{1-6}$ alkoxy, and $C_{2-6}$ alkynyl, wherein the $C_{1-6}$ alkyl, $C_{1-6}$ alkoxy, and $C_{2-6}$ alkynyl are each independently and optionally substituted with one or more deuterium atoms or halogens;
m is selected from the group consisting of 0, 1, 2, 3, and 4;
$L_1$ is $C_{1-3}$ alkylene, wherein the $C_{1-3}$ alkylene is substituted with one or more $R^B$, and $R^B$ is $C_{1-6}$ alkyl, wherein the $C_{1-6}$ alkyl is substituted with one or more $R^{1B}$, and $R^{1B}$ is hydroxy;
$R^2$, $R^3$, $R^4$, $R^5$, and $R^6$ are each independently selected from the group consisting of hydrogen and deuterium;
$R^7$ is $C_{1-3}$ alkyl, and the $C_{1-3}$ alkyl is optionally substituted with one or more deuterium atoms;
$L_2$ is selected from the group consisting of NH, O, and $C_{1-3}$ alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl;
ring A is selected from the group consisting of phenyl and 5- to 6-membered heteroaryl;
$R^8$ is selected from the group consisting of deuterium, halogen, hydroxy, cyano, carboxyl, $C_{1-6}$ alkyl, $C_{1-6}$ alkoxy, $C_{2-6}$ alkenyl, $C_{2-6}$ alkynyl, 3- to 6-membered cycloalkyl, -S-$C_{1-6}$ alkyl, and -NHR'R", and R' and R" are each independently selected from the group consisting of hydrogen and $C_{1-6}$ alkyl, wherein the $C_{1-6}$ alkyl, $C_{1-6}$ alkoxy, $C_{2-6}$ alkenyl, $C_{2-6}$ alkynyl, and 3- to 6-membered cycloalkyl are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;
n is selected from the group consisting of 0, 1, 2, 3, and 4;
$R^9$ is hydrogen or deuterium;
$L_3$ is $C_{1-3}$ alkylene, wherein the $C_{1-3}$ alkylene is optionally substituted with one or more $R^E$, and $R^E$ is deuterium;
$R^{10}$ and $R^{11}$ are each independently $C_{1-3}$ alkyl, wherein the $C_{1-3}$ alkyl is optionally substituted with one or more $R^F$, and $R^F$ is deuterium.

**4.** The compound or the pharmaceutically acceptable salt thereof according to any one of claims 1-3, wherein $L_2$ is NH.

**5.** The compound or the pharmaceutically acceptable salt thereof according to any one of claims 1-3, wherein $L_2$ is O.

**6.** The compound or the pharmaceutically acceptable salt thereof according to any one of claims 1-3, wherein $L_2$ is $C_{1-3}$

alkylene, wherein the $C_{1-3}$ alkylene is optionally substituted with one or more $R^C$, and $R^C$ is selected from the group consisting of deuterium, halogen, hydroxy, cyano, $C_{1-6}$ alkyl, and $C_{1-6}$ haloalkyl; preferably, $L_2$ is methylene, wherein the methylene is optionally substituted with one or more $R^C$, and $R^C$ is selected from the group consisting of deuterium, halogen, hydroxy, cyano, $C_{1-6}$ alkyl, and $C_{1-6}$ haloalkyl; more preferably, $L_2$ is methylene.

**7.** The compound or the pharmaceutically acceptable salt thereof according to any one of claims 1-3, being a compound represented by formula (II) or a pharmaceutically acceptable salt thereof:

(II)

wherein $R^1$, m, $L_1$, $R^2$, $R^3$, $R^4$, $R^5$, $R^6$, ring A, $R^8$, n, $L_3$, $R^{10}$, and $R^{11}$ are as defined in claim 1 or 2.

**8.** The compound or the pharmaceutically acceptable salt thereof according to any one of claims 1 to 7, wherein $L_3$ is selected from the group consisting of methylene and ethylene, wherein the methylene and ethylene are each independently and optionally substituted with one or more $R^E$, and $R^E$ is deuterium;
preferably, $L_3$ is methylene, wherein the methylene is optionally substituted with one or more $R^E$, and $R^E$ is deuterium.

**9.** The compound or the pharmaceutically acceptable salt thereof according to any one of claims 1 to 8, wherein $R^{10}$ is $C_{1-3}$ alkyl, wherein the $C_{1-3}$ alkyl is optionally substituted with one or more $R^F$, and $R^F$ is deuterium; preferably, $R^{10}$ is selected from the group consisting of methyl and ethyl, wherein the methyl and ethyl are each independently and optionally substituted with one or more $R^F$, and $R^F$ is deuterium; more preferably, $R^{10}$ is selected from the group consisting of methyl and $-CD_3$.

**10.** The compound or the pharmaceutically acceptable salt thereof according to any one of claims 1 to 9, wherein $R^{11}$ is $C_{1-3}$ alkyl, wherein the $C_{1-3}$ alkyl is optionally substituted with one or more $R^F$, and $R^F$ is deuterium; preferably, $R^{11}$ is selected from the group consisting of methyl and ethyl, wherein the methyl and ethyl are each independently and optionally substituted with one or more $R^F$, and $R^F$ is deuterium; more preferably, $R^{11}$ is selected from the group consisting of methyl and $-CD_3$.

**11.** The compound or the pharmaceutically acceptable salt thereof according to any one of claims 1 to 10, wherein ring A is phenyl.

**12.** The compound or the pharmaceutically acceptable salt thereof according to any one of claims 1 to 10, wherein ring A is 5- to 6-membered heteroaryl, preferably pyridine, pyrazole, imidazole, or thiazole.

**13.** The compound or the pharmaceutically acceptable salt thereof according to any one of claims 1, 2, and 4 to 12, wherein $L_1$ is a linking bond.

**14.** The compound or the pharmaceutically acceptable salt thereof according to any one of claims 1, 2, and 4 to 12, wherein $L_1$ is $C_{1-3}$ alkylene, wherein the $C_{1-3}$ alkylene is optionally substituted with one or more $R^B$, and $R^B$ is selected from the group consisting of deuterium, halogen, hydroxy, oxo, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium, halogen, $C_{1-6}$ alkyl, $C_{1-6}$ haloalkyl, and deuterated $C_{1-6}$ alkyl;

preferably, $L_1$ is methylene, wherein the methylene is substituted with one or more $R^B$, and $R^B$ is selected from the group consisting of deuterium, halogen, $C_{1-6}$ alkyl, 3- to 6-membered cycloalkyl, and 3- to 6-membered heterocycloalkyl, wherein the $C_{1-6}$ alkyl, 3- to 6-membered cycloalkyl, and 3- to 6-membered heterocycloalkyl

are each independently and optionally substituted with one or more $R^{1B}$, and $R^{1B}$ is selected from the group consisting of deuterium, halogen, $C_{1-6}$ alkyl, $C_{1-6}$ haloalkyl, and deuterated $C_{1-6}$ alkyl;
more preferably, $L_1$ is methylene, wherein the methylene is substituted with one or more $R^B$, and $R^B$ is selected from the group consisting of deuterium, halogen, $C_{1-3}$ alkyl, 3- to 6-membered cycloalkyl, and 3- to 6-membered heterocycloalkyl, wherein the $C_{1-3}$ alkyl, 3- to 6-membered cycloalkyl, and 3- to 6-membered heterocycloalkyl are each independently and optionally substituted with one or more $R^{1B}$, and $R^{1B}$ is selected from the group consisting of deuterium and halogen.

**15.** The compound or the pharmaceutically acceptable salt thereof according to any one of claims 7 to 11 and 14, wherein ring A is phenyl;

$R^2$, $R^3$, $R^4$, $R^5$, and $R^6$ are each independently hydrogen;
$R^{10}$ is selected from the group consisting of methyl and $-CD_3$;
$R^{11}$ is selected from the group consisting of methyl and $-CD_3$;
$L_1$ is methylene, wherein the methylene is substituted with one or more $R^B$, and $R^B$ is selected from the group consisting of deuterium, halogen, methyl, ethyl, cyclopropyl, and cyclobutyl, wherein the methyl, ethyl, cyclopropyl, and cyclobutyl are each independently and optionally substituted with one or more $R^{1B}$, and $R^{1B}$ is selected from the group consisting of deuterium and halogen.

**16.** The compound or the pharmaceutically acceptable salt thereof according to any one of claims 1 to 15, wherein $R^8$ is selected from the group consisting of deuterium, halogen, hydroxy, cyano, carboxyl, $C_{1-6}$ alkyl, and $C_{1-6}$ alkoxy, wherein the $C_{1-6}$ alkyl and $C_{1-6}$ alkoxy are each independently and optionally substituted with one or more $R^D$, and $R^D$ is selected from the group consisting of deuterium, halogen, hydroxy, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more deuterium atoms or halogens;

preferably, $R^8$ is selected from the group consisting of deuterium, halogen, hydroxy, cyano, carboxyl, methyl, ethyl, isopropyl, n-propyl, n-butyl, methoxy, ethoxy, isopropoxy, and n-butoxy, wherein the methyl, ethyl, isopropyl, n-propyl, n-butyl, methoxy, ethoxy, isopropoxy, and n-butoxy are each independently and optionally substituted with one or more $R^D$, and $R^D$ is selected from the group consisting of deuterium, halogen, hydroxy, methyl, ethyl, ethoxy, and methoxy, wherein the methyl, ethyl, ethoxy, and methoxy are each independently and optionally substituted with one or more deuterium atoms or halogens;
more preferably, $R^8$ is selected from the group consisting of deuterium, halogen, methyl, ethyl, methoxy, and ethoxy, wherein the methyl, ethyl, methoxy, and ethoxy are each independently and optionally substituted with one or more $R^D$, and $R^D$ is selected from the group consisting of deuterium, halogen, and hydroxy;
n is selected from the group consisting of 0, 1, 2, and 3.

**17.** The compound or the pharmaceutically acceptable salt thereof according to any one of claims 1 to 16, wherein $R^1$ is selected from the group consisting of deuterium, cyano, $C_{1-6}$ alkyl, $C_{1-6}$ alkoxy, 3- to 6-membered cycloalkyl, and 3- to 6-membered heterocycloalkyl, wherein the $C_{1-6}$ alkyl, $C_{1-6}$ alkoxy, 3- to 6-membered cycloalkyl, and 3- to 6-membered heterocycloalkyl are each independently and optionally substituted with one or more $R^A$, and $R^A$ is selected from the group consisting of deuterium, halogen, hydroxy, amino, oxo, $C_{1-6}$ alkyl, and $C_{1-6}$ alkoxy, wherein the $C_{1-6}$ alkyl and $C_{1-6}$ alkoxy are each independently and optionally substituted with one or more deuterium atoms or halogens;

preferably, $R^1$ is selected from the group consisting of $C_{1-6}$ alkyl and 3- to 6-membered cycloalkyl, wherein the $C_{1-6}$ alkyl and 3- to 6-membered cycloalkyl are each independently and optionally substituted with one or more $R^A$, and $R^A$ is selected from the group consisting of deuterium, halogen, hydroxy, amino, oxo, methyl, ethyl, methoxy, and ethoxy;
more preferably, $R^1$ is selected from the group consisting of methyl, ethyl, n-propyl, isopropyl, n-butyl, isobutyl, cyclopropyl, cyclobutyl, and cyclopentyl, wherein the methyl, ethyl, n-propyl, isopropyl, n-butyl, isobutyl, cyclopropyl, cyclobutyl, and cyclopentyl are each independently and optionally substituted with one or more $R^A$, and $R^A$ is selected from the group consisting of deuterium, halogen, and hydroxy;
m is selected from the group consisting of 0, 1, 2, and 3.

**18.** The compound or the pharmaceutically acceptable salt thereof according to any one of claims 1, 2, 4 to 12, and 14 to 17, wherein $L_1$ is methylene, wherein the methylene is substituted with one or more $R^B$, and $R^B$ is deuterium or $C_{1-6}$ alkyl.

**19.** The compound or the pharmaceutically acceptable salt thereof according to any one of claims 1, 2, 4 to 12, and 14 to 18, wherein

is selected from the group consisting of

,  ,  ,

,  ,  ,  ,

and

.

**20.** The compound or the pharmaceutically acceptable salt thereof according to any one of claims 1, 2, 4, 7 to 11, and 14 to 19, being a compound represented by formula (III-1), (III-2), (III-3), or (III-4) or a pharmaceutically acceptable salt thereof:

(III-1) ,

(III-2) ,

(III-3) , or

(III-4) ,

wherein $R^8$, n, $R^1$, and m are as defined in claim 1; $R^B$ is selected from the group consisting of hydrogen, deuterium, methyl, ethyl, cyclopropyl, and cyclobutyl, wherein the methyl, ethyl, cyclopropyl, and cyclobutyl are each indepen-

dently and optionally substituted with one or more $R^{1B}$, and $R^{1B}$ is selected from the group consisting of deuterium and halogen.

**21.** The compound or the pharmaceutically acceptable salt thereof according to claim 20, wherein $R^8$ is selected from the group consisting of deuterium, halogen, methyl, ethyl, methoxy, and ethoxy, wherein the methyl, ethyl, methoxy, and ethoxy are each independently and optionally substituted with one or more $R^D$, and $R^D$ is selected from the group consisting of deuterium, halogen, and hydroxy;

$R^1$ is selected from the group consisting of methyl, ethyl, n-propyl, isopropyl, n-butyl, isobutyl, cyclopropyl, cyclobutyl, and cyclopentyl, wherein the methyl, ethyl, n-propyl, isopropyl, n-butyl, isobutyl, cyclopropyl, cyclobutyl, and cyclopentyl are each independently and optionally substituted with one or more $R^A$, and $R^A$ is selected from the group consisting of deuterium and halogen;
m and n are each independently selected from the group consisting of 0, 1, 2, and 3.

**22.** The compound or the pharmaceutically acceptable salt thereof according to any one of claim 20 or 21, wherein:

$R^B$ is selected from the group consisting of methyl and cyclopropyl, wherein the methyl and cyclopropyl are each independently and optionally substituted with one or more $R^{1B}$, and $R^{1B}$ is deuterium;
$R^1$ is selected from the group consisting of methyl and cyclopropyl, wherein the methyl and cyclopropyl are each independently and optionally substituted with one or more $R^A$, and $R^A$ is selected from the group consisting of deuterium and halogen;
m is selected from the group consisting of 0, 1, 2, and 3;
n is 0.

**23.** The compound or the pharmaceutically acceptable salt thereof according to claim 1, being selected from the group consisting of the following compounds or pharmaceutically acceptable salts thereof:

, , , ,

, , and ,

preferably the following compounds or pharmaceutically acceptable salts thereof:

, , ,

, , ,

, , ,

, , ,

, , , and .

24. An isotopically substituted form of the compound according to any one of claims 1-23, wherein preferably, the isotopically substituted form is a deuterated form.

25. A preparation method for the compound or the pharmaceutically acceptable salt thereof according to any one of claims 1 to 24:

(I-A) + (I-B) →

,

comprising a step of catalyzing a reaction of a compound represented by formula (I-A) or a pharmaceutically acceptable salt thereof with a compound represented by formula (I-B) or a pharmaceutically acceptable salt thereof in an alkaline environment with a catalyst selected from the group consisting of carbonyldiimidazole, phosgene, and triphosgene,

wherein $L_2$ is NH;
$R^1$, m, $L_1$, $R^2$, $R^3$, $R^4$, $R^5$, $R^6$, $R^7$, ring A, $R^8$, n, $R^9$, $L_3$, $R^{10}$, and $R^{11}$ are as defined in any one of claims 1 to 22.

26. A compound represented by formula (I-A) or a pharmaceutically acceptable salt thereof:

(I-A)

wherein $R^1$, m, $L_1$, $R^2$, $R^3$, $R^4$, $R^5$, $R^6$, and $R^7$ are as defined in any one of claims 1-3.

27. A pharmaceutical composition, comprising the compound or the pharmaceutically acceptable salt thereof according to any one of claims 1 to 23 or the isotopically substituted form according to claim 24, and a pharmaceutically acceptable excipient.

28. Use of the compound or the pharmaceutically acceptable salt thereof according to any one of claims 1 to 23, the isotopically substituted form according to claim 24, or the pharmaceutical composition according to claim 27 in the manufacture of a medicament for treating and/or preventing a disease or disorder associated with abnormal activity of a serine/threonine kinase.

29. Use of the compound or the pharmaceutically acceptable salt thereof according to any one of claims 1 to 23, the isotopically substituted form according to claim 24, or the pharmaceutical composition according to claim 27 in the

manufacture of a medicament for treating and/or preventing a disease or disorder associated with abnormal activity of CDK7, wherein preferably, the disease or disorder associated with abnormal activity of CDK7 is selected from the group consisting of a proliferative disease, an inflammatory disease, an auto inflammatory disease, an autoimmune disease, and an infectious disease.

**30.** Use of the compound or the pharmaceutically acceptable salt thereof according to any one of claims 1 to 23, the isotopically substituted form according to claim 24, or the pharmaceutical composition according to claim 27 in the manufacture of a medicament for treating and/or preventing a disease or disorder, wherein the disease or disorder is selected from the group consisting of a proliferative disease, an inflammatory disease, an autoinflammatory disease, an autoimmune disease, and an infectious disease.

**31.** The use according to any one of claim 29 or 30, wherein the proliferative disease is cancer; preferably, the cancer is selected from the group consisting of a hematological tumor and a solid tumor, wherein the hematological tumor is selected from the group consisting of chronic lymphocytic leukemia, acute lymphocytic leukemia, T-cell acute lymphocytic leukemia, chronic myeloid leukemia, and acute myeloid leukemia, and the solid tumor is selected from the group consisting of breast cancer, intestinal cancer, lung cancer, pancreatic cancer, prostate cancer, Ewing sarcoma, osteoma, neuroblastoma, cervical cancer, ovarian cancer, gastric cancer, and liver cancer.

**32.** The use according to claim 31, wherein the breast cancer is triple-negative breast cancer or ER/PR+ HER2- breast cancer, and preferably, the ER/PR+ HER2- breast cancer is ER/PR+ HER2- breast cancer resistant to a CDK4/6 inhibitor; the lung cancer is selected from the group consisting of non-small cell lung cancer and small cell lung cancer; the intestinal cancer is selected from the group consisting of colon cancer and rectal cancer.

**33.** Use of the compound or the pharmaceutically acceptable salt thereof according to any one of claims 1 to 23 or the isotopically substituted form according to claim 24 in the manufacture of an antibody-drug conjugate or a proteolysis targeting chimera.

**34.** An antibody-drug conjugate, comprising the compound or the pharmaceutically acceptable salt thereof according to any one of claims 1 to 23 or the isotopically substituted form according to claim 24.

**35.** A proteolysis targeting chimera, comprising the compound or the pharmaceutically acceptable salt thereof according to any one of claims 1 to 23 or the isotopically substituted form according to claim 24.

## INTERNATIONAL SEARCH REPORT

International application No. **PCT/CN2024/126642**

**A. CLASSIFICATION OF SUBJECT MATTER**

C07D471/04(2006.01)i; C07D407/04(2006.01)i; A61K31/35(2006.01)i; A61K31/41(2006.01)i; A61K31/435(2006.01)i; A61K31/4375(2006.01)i; A61K31/495(2006.01)i; A61K31/519(2006.01)i; A61K31/535(2006.01)i; A61P35/00(2006.01)i; A61P37/00(2006.01)i

According to International Patent Classification (IPC) or to both national classification and IPC

**B. FIELDS SEARCHED**

Minimum documentation searched (classification system followed by classification symbols)

C07D471/-,C07D407/-,A61K31/-,A61P35/-,A61P37/-

Documentation searched other than minimum documentation to the extent that such documents are included in the fields searched

Electronic data base consulted during the international search (name of data base and, where practicable, search terms used)

CNKI, VEN, DWPI, CNTXT, CNABS, VCN, ENTXTC, STN(REG, CAPLUS, CASREACT): 拓界生物, 朱国栋, 乔彭, 李云飞, 噻唑, 抑制, 哌啶, 嘧啶, CDK, 癌, 肿瘤, 细胞周期蛋白激酶, thiazole, piperidin+, pyrimidin+, inhibit+, tumour, tumor, cancer.

**C. DOCUMENTS CONSIDERED TO BE RELEVANT**

| Category* | Citation of document, with indication, where appropriate, of the relevant passages | Relevant to claim No. |
|---|---|---|
| PX | WO 2024088296 A1 (TUOJIE BIOTECH (SHANGHAI) CO., LTD.) 02 May 2024 (2024-05-02)<br>claims 1-38 | 1-35 |
| X | CN 103635472 A (ARRAY BIOPHARMA, INC. et al.) 12 March 2014 (2014-03-12)<br>claims 1-29, and description, pages 31-93, and embodiment 1 | 1-35 |
| A | CN 116194103 A (JANSSEN PHARMACEUTICA N.V.) 30 May 2023 (2023-05-30)<br>entire document | 1-35 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 January 2025** | **16 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/126642**

| Patent document cited in search report | Publication date (day/month/year) | Patent family member(s) | Publication date (day/month/year) |
|---|---|---|---|
| WO 2024088296 A1 | 02 May 2024 | TW 202421138 A | 01 June 2024 |
| CN 103635472 A | 12 March 2014 | ES 2543050 T3 | 14 August 2015 |
| | | EP 2681215 A1 | 08 January 2014 |
| | | EP 2681215 B1 | 22 April 2015 |
| | | BR 112013021896 A2 | 08 November 2016 |
| | | RU 2013143839 A | 10 April 2015 |
| | | MX 2013009877 A | 11 February 2014 |
| | | MX 339873 B | 15 June 2016 |
| | | KR 20140014190 A | 05 February 2014 |
| | | KR 101961500 B1 | 22 March 2019 |
| | | CA 2828478 A1 | 07 September 2012 |
| | | CA 2828478 C | 31 December 2019 |
| | | JP 2014506930 A | 20 March 2014 |
| | | JP 6085866 B2 | 01 March 2017 |
| | | US 2013338140 A1 | 19 December 2013 |
| | | US 9133187 B2 | 15 September 2015 |
| | | WO 2012118850 A1 | 07 September 2012 |
| CN 116194103 A | 30 May 2023 | AU 2021350161 A1 | 08 June 2023 |
| | | AU 2021350161 A9 | 08 February 2024 |
| | | BR 112023005005 A2 | 18 April 2023 |
| | | KR 20230074762 A | 31 May 2023 |
| | | US 2024101554 A1 | 28 March 2024 |
| | | MX 2023003516 A | 19 April 2023 |
| | | EP 4217356 A1 | 02 August 2023 |
| | | JP 2023542411 A | 06 October 2023 |
| | | CA 3191993 A1 | 31 March 2022 |
| | | WO 2022064009 A1 | 31 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016058544 A
- WO 2018013867 A
- WO 2019143719 A
- WO 2019143730 A
- WO 2019099298 A
- WO 2020093006 A
- WO 2020093011 A
- WO 2022064009 A